(19) **Europäisches Patentamt European Patent Office Office européen des brevets**

(11) **EP 3 513 355 B1**

(12) **EUROPÄISCHE PATENTSCHRIFT**

(45) Veröffentlichungstag und Bekanntmachung des Hinweises auf die Patenterteilung:
**29.04.2026 Patentblatt 2026/18**

(21) Anmeldenummer: **17777502.0**

(22) Anmeldetag: **15.09.2017**

(51) Internationale Patentklassifikation (IPC):
**G06N 3/09** *(2023.01)* **G06N 3/0499** *(2023.01)*
**G01N 21/65** *(2006.01)* **G01N 21/64** *(2006.01)*
**G01J 3/28** *(2006.01)* **G01N 33/08** *(2006.01)*
**G06V 20/69** *(2022.01)* **G06F 18/25** *(2023.01)*

(52) Gemeinsame Patentklassifikation (CPC):
**G01J 3/28; G01N 33/085; G06F 18/25;**
**G06N 3/0499; G06N 3/09;** G01N 21/6486;
G01N 21/65; G01N 2201/1293; G06F 2218/12

(86) Internationale Anmeldenummer:
**PCT/EP2017/073236**

(87) Internationale Veröffentlichungsnummer:
**WO 2018/050802 (22.03.2018 Gazette 2018/12)**

(54) **VERFAHREN ZUR KLASSIFIZIERUNG VON SPEKTREN VON OBJEKTEN MIT KOMPLEXEM INFORMATIONSGEHALT**

METHOD FOR CLASSIFYING SPECTRA OF OBJECTS HAVING COMPLEX INFORMATION CONTENT

PROCÉDÉ DE CLASSIFICATION DE SPECTRES D'OBJETS À CONTENU D'INFORMATION COMPLEXE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.09.2016 DE 102016011348**

(43) Veröffentlichungstag der Anmeldung:
**24.07.2019 Patentblatt 2019/30**

(73) Patentinhaber: **Technische Universität Dresden**
**01069 Dresden Sachsen (DE)**

(72) Erfinder:
• STEINER, Gerald
  08340 Schwarzenberg (DE)
• PREUSSE, Grit
  01445 Radebeul (DE)
• KOCH, Edmund
  01307 Dresden (DE)
• GALLI, Roberta
  01309 Dresden (DE)
• SCHNABEL, Christian
  01099 Dresden (DE)

• PREUSSE, Johanna
  01455 Radebeul (DE)

(74) Vertreter: **Rauschenbach, Marion**
**Rauschenbach + Schied Patentanwälte PartG mbB**
**Bertolt-Brecht-Allee 22**
**01309 Dresden (DE)**

(56) Entgegenhaltungen:
**DE-A1- 102008 040 838    US-A1- 2008 025 591**

• XU L ET AL: "Ensemble preprocessing of near-infrared (NIR) spectra for multivariate calibration", ANALYTICA CHIMICA ACTA, ELSEVIER, AMSTERDAM, NL, vol. 616, no. 2, 2 June 2008 (2008-06-02), pages 138 - 143, XP022659665, ISSN: 0003-2670, [retrieved on 20080420], DOI: 10.1016/J.ACA.2008.04.031

- **ELADIO RODRIGUEZ-DIAZ ET AL: "Spectral classifier design with ensemble classifiers and misclassificationrejection: application to elastic-scattering spectroscopy for detection of colonic neoplasia", JOURNAL OF BIOMEDICAL OPTICS, vol. 16, no. 6, 1 January 2011 (2011-01-01), 1000 20th St. Bellingham WA 98225-6705 USA, pages 067009, XP055345782, ISSN: 1083-3668, DOI: 10.1117/1.3592488**

**Beschreibung**

Die Erfindung betrifft ein Verfahren zur Klassifizierung von Spektren von Objekten mit komplexem Informationsgehalt mit mindestens zwei unterschiedlichen Objektinformationen, insbesondere von optischen Molekülspektren zur Zuordnung der Objektinformationen.

Bekannt sind Druckschriften zu Verfahren zur gestützten Klassifizierung optischer Spektren. Dazu gehören u.a. die Druckschrift A. E. Nikulin, B. Dolenko, T., Bezabeh, R. L. Somorjai: Near-optimal region selection for feature space reduction: novel preprocessing methods for classifying MR spectra, NMR Biomed. 11 (4-5), 1998, S. 209-216, die Druckschrift B. K. Lavine, C. E. Davidson, A. J. Moores: Genetic algorithms for spectral pattern recognition, Vibrational Spectroscopy, Volume 28, Issue 1, 2002, Pages 83-95, wobei darin der Algorithmus auf den Hauptkomponenten basiert und eine Wichtung spektraler Bereiche zur Klassifizierung herangezogen wird, sowie die Druckschrift J. Jacques, C. Bouveyron, S. Girard, O. Devos, L. Duponchel, C. Ruckebusch: Gaussian mixture models for the classification of high-dimensional vibrational spectroscopy data, Journal of Chemometrics, Volume 24, Issue 11-12, S. 719-727 (Dez. 2010).

Darin wird ein Verfahren beschrieben, bei dem besonders hochdimensionale spektrale Daten in sogenannte Teilräume (engl. subspaces) zerlegt werden, die nachfolgend mittels Diskriminanzanalyse klassifiziert werden.

Bekannt sind auch Eladio Rodriguez-Diaz, Satish K. Singh, Irving J. Bigio, David A. Castañón, "Spectral classifier design with ensemble classifiers and misclassificationrejection: application to elastic-scattering spectroscopy for detection of colonic neoplasia," J. Biomed. Opt. 16(6) 067009 (1 June 2011), das verschiedene Spektralbereiche separat klassifiziert und ihre Ergebnisse mithilfe eines Ensemble-Systems kombiniert; sowie Lu Xu, Yan-Ping Zhou, Li-Juan Tang, Hai-Long Wu, Jian-Hui Jiang, Guo-Li Shen, Ru-Qin Yu, Ensemble preprocessing of near-infrared (NIR) spectra for multivariate calibration, Analytica Chimica Acta, Volume 616, Issue 2, 2008, Pages 138-143, ISSN 0003-2670, das mehrere Vorverarbeitungen von NIR-Spektren parallel anwendet und die daraus entstehenden Kalibrationsmodelle in einem Ensemble kombiniert, um robustere und genauere multivariate Kalibrationsmodelle zu erhalten und so die Abhängigkeit von einer einzelnen "besten" Vorverarbeitungs-Methode zu reduzieren.

Optische Molekülspektren weisen einen großen Informationsgehalt über die molekularen Eigenschaften des untersuchten Objektes auf. Insbesondere Schwingungsspektren gelten aufgrund ihrer hohen Informationsdichte über die molekulare Struktur als Fingerabdruck von Molekülen. Bei der spektroskopischen Analyse komplexer biologischer Objekte müssen die entsprechend der Vorgabe relevanten Informationen von den weniger oder nicht bedeutsamen Informationen als auch von Störungen separiert werden. Dazu werden üblicherweise Verfahren der Chemometrik, bei höherdimensionalen Daten auch multivariate Verfahren eingesetzt.

Sind wichtige spektrale Merkmale der gesuchten molekularen Information von Objekten bekannt, können gestützte (supervidierte) Klassifikationsverfahren eingesetzt werden, wie beispielsweise in der Druckschrift G. Steiner, S. Kuchler, A. Herrmann, E. Koch, R. Salzer, G. Schackert, M. Kirsch: Cytometry, Part A 2008, 73A, 1158-1164 beschrieben.

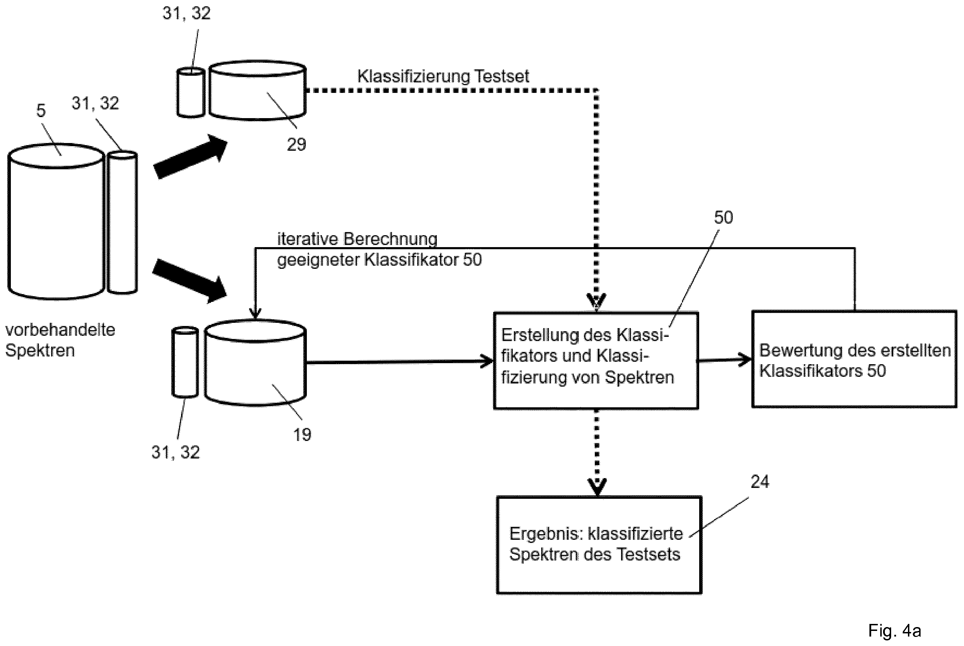

Die gestützten Klassifikationsverfahren zeichnen sich gegenüber anderen Verfahren durch eine höhere Genauigkeit in der Erkennung und quantitativen Bewertung der gesuchten Informationen aus. Bei dem bekannten Verfahren der gestützten Klassifizierung gemäß Fig. 4a wird mittels eines Trainingsset 19, das aus repräsentativen Spektren mit zugeordneten Eigenschaften besteht, ein Klassifikator 50 berechnet. Der Klassifikator 50 wird dann anhand eines unabhängigen Testsets 29, d.h. die Spektren werden nicht zum Aufbau des Klassifikators 50 eingesetzt, wie in Fig. 4a gezeigt, zur Validierung überprüft und bewertet und ein klassifiziertes Testset 24 erreicht.

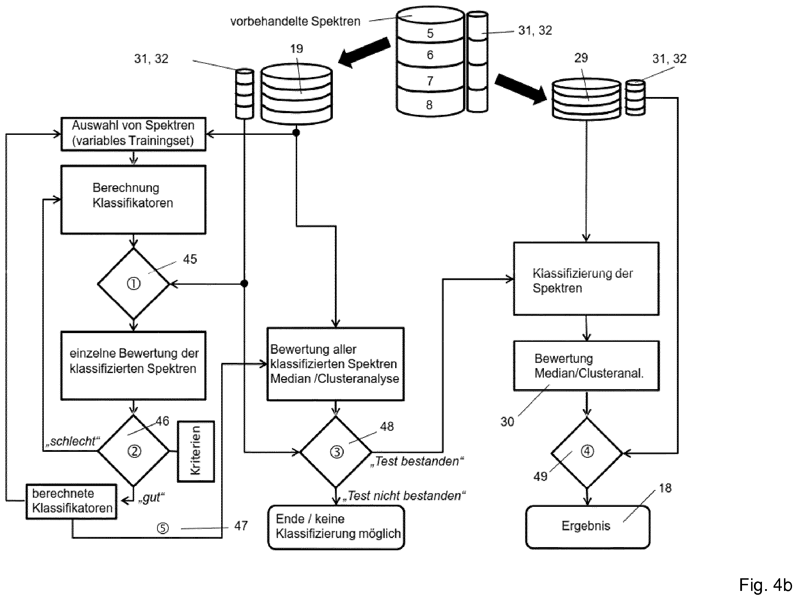

Der Aufbau des Klassifikators 50 mittels des Trainingssets 19 wird durch ein Testset 29 mit z.B. maximal 30% der Spektren (gestrichelte Linie zu dem erstellten Klassifikator) gemäß Fig. 4b verifiziert, so dass im Ergebnis ein klassifiziertes Testset 24 (gestrichelte Linie von dem Klassifikator 50) zum klassifizierten Testset 24 erreicht wird.

Ein generelles Problem der gestützten Klassifizierungsverfahren ist die Abwägung zwischen Genauigkeit der erzielten Zuordnungen und Robustheit der Klassifizierung. Häufig lassen sich sehr hohe Genauigkeiten nur bei einem sogenannten Übertraining des Klassifikators erzielen. Darunter wird verstanden, dass der Klassifikator nur bestimmte Spektren noch richtig zuordnen kann, wobei dies mit sehr hoher Genauigkeit geschieht. Dagegen führen bereits geringste Abweichungen oder Störeinflüsse zu einer dramatisch reduzierten Genauigkeit der Klassifikation. Es wird daher eine Anpassung zwischen bestmöglicher Klassifizierung und hoher Robustheit der Klassifizierung angestrebt.

Liegen Spektren mit sehr hoher Variabilität vor, wie zum Beispiel bei in-ovo Spektren zur Geschlechtsbestimmung von Hühnereiern, müssen zwangsläufig für die Bewahrung einer ausreichenden Robustheit der Klassifizierung Abstriche bei der Genauigkeit vorgenommen werden. Grundsätzlich lässt sich dieser immanente Widerspruch nicht lösen. Um dennoch eine gute Stabilität bei ausreichender Genauigkeit zu erzielen, wurden in den zurückliegenden Jahren verschiedenen Verfahren zur Klassifizierung neu entwickelt. Die grundsätzliche Herangehensweise ist dabei die Parallelisierung der Klassifizierung über verschiedene Entscheidungsbäume. Das Random Forest Verfahren (engl. Random Forest Methode) basiert auf einem Netz von unkorrelierten Entscheidungsbäumen, wobei die Entscheidungsbäume durch Randomisierung während des Trainingsprozesses gewachsen sind bzw. verknüpft werden. Jeder sogenannte Baum trifft eine Entscheidung. Die Gruppe der Bäume mit den meisten, jeweils identischen Entscheidungen, bestimmt das Ergebnis der Klassifizierung, also die Zuordnung des Spektrums. Allerdings kann das Random Forest Verfahren nicht auf unterschiedlich auftretende Störungen oder Variationen in den Spektren reagieren. Auch hier kann allein durch das Aufstellen von zu vielen Bäumen ein Übertraining auftreten.

In der Schrift US20120321174 A1 wird ein Klassifikationsverfahren, beruhend auf dem Random Forest Verfahren für die Bildauswertung, beschrieben. Dieses gestützte Klassifizierungsverfahren ist so gestaltet, dass insbesondere kleine, aber relevante Merkmale für die Klassifizierung berücksichtigt werden.

Diese relevanten Merkmale des allgemeinen Klassifizierungsverfahrens können auch z.B. in der in-ovo Spektroskopie von Hühnereiern in Form von kleinen auf die Spektren bezogenen Signale der Geschlechtsinformationen definiert werden und eine Rolle spielen.

Bei der in-ovo Spektroskopie von Hühnereiern wird jeweils ein gestütztes Klassifizierungsverfahren zur Identifizierung des Geschlechts eingesetzt.

Optische in-ovo Spektren zeichnen sich aber oftmals durch eine sehr hohe natürliche Variabilität aus, die die vergleichsweise kleinen Signale der Geschlechtsinformation deutlich überlagern. Hinzu kommen nicht vermeidbare externe Einflüsse von der Umgebung der Messung selbst.

Derzeit werden folgende unterschiedliche Verfahren zur Klassifizierung der Spektren von Objekten, insbesondere zur Geschlechtsbestimmung von befruchteten und/oder bebrüteten Eiern in den unten genannten Druckschriften angegeben:

In der Druckschrift WO 2010/150265 A wird ein Verfahren, beruhend auf einer Färbung, insbesondere der Federn des entwickelten Embryos beschrieben. Das Verfahren beruht darauf, dass im fortgeschrittenen Entwicklungsstadium (Tag 12 der Bebrütung) die Farbe der Federn bei bestimmten Hühnerrassen Rückschluss auf das Geschlecht zulässt. Die Auswertung erfolgt mit einem Algorithmus zur Klassifizierung.

Des Weiteren wird in der Druckschrift WO 2014/021715 A2 ein Verfahren beschrieben, bei dem das Geschlecht des Embryos mittels endokrinologischer Analyse bestimmt wird.

Die Druckschrift DE 10 2007 013 107 A1 beschreibt die Anwendung der Ramanspektroskopie zur Geschlechtsbestimmung von Vögeln, wobei allgemein zellhaltiges Material untersucht wird. Es wird jedoch kein Verfahren zur in-ovo Geschlechtsbestimmung beschrieben.

Die Molekülspektren werden mittels Verfahren und Vorrichtungen gemäß den nachfolgend genannten Druckschriften registriert:

Ein Verfahren und Vorrichtungen zur Bestimmung des Geschlechtes von Hühnereiern, basierend auf der optischen, vorzugsweise fasergekoppelten Spektroskopie werden in der Druckschrift DE 10 2010 006 161 B3 beschrieben. Es werden jedoch keine Verfahren zur Analyse der Spektren und zur Klassifizierung beschrieben

In den Druckschriften DE 10 2014 010 150 A1 und WO 2016/000678 A1 werden Verfahren und Vorrichtungen zur Ramanspektroskopischen in ovo Geschlechtsbestimmung beschrieben. Die Auswertung der Spektren kann vorteilhaft mit chemometrischen Verfahren erfolgen.

Die Druckschrift EP 2 336 751 A1 beschreibt ein Verfahren zur Bestimmung des Geschlechts von Vogeleiern. Bei dem Verfahren wird die Keimscheibe eines Eies mit Licht beleuchtet und die emittierte Fluoreszenz zeitaufgelöst erfasst. Die Erkennung des Geschlechtes geschieht mit Hilfe einer gestützten Klassifizierung, wobei mittels des Verfahrens der fraktalen Dimension ein Klassifikator berechnet wird.

In der Schrift US 6 029 080 B wird ein Verfahren zur in ovo Geschlechtsbestimmung beschrieben. Aus der Analyse von MRT-Bildern des Eies lassen sich ab einem bestimmten Entwicklungsstadium des Embryos die Geschlechtsorgane erkennen und für die Geschlechtsbestimmung heranziehen.

Der Nachteile in der Auswertung dieser Verfahren besteht darin, dass jedes dieser Verfahren letztendlich für sich ein Verfahren zur Klassifizierung von Spektren von Objekten mit nur einem Klassifikator nutzt.

Zu den Nachteilen ist zusammenfassend zu bemerken, dass, um die gesuchten Geschlechtsinformationen dennoch sicher aus den registrierten Spektren zu extrahieren, deshalb die Verwendung nur eines einzigen Klassifikators nicht ausreichend ist, um die Erkennungssicherheit der bestimmten Geschlechtsinformationen als ausreichend zu betrachten. Vielmehr müssen die variablen Einflüsse und die Variationen in der biochemischen Zusammensetzung des Eies als auch in den unterschiedlichen Entwicklungsstadien berücksichtigt werden. Um diese große Variationsbreite in ein erkennungssicheres Verfahren zur Klassifizierung einzubeziehen, wird eingeschätzt, dass deshalb die Berechnung nur eines Klassifikators nicht ausreichen wird.

Der Erfindung liegt deshalb die Aufgabe zugrunde, ein Verfahren zur Klassifizierung von Spektren von Objekten mit komplexem Informationsgehalt anzugeben, das derart geeignet ausgebildet ist, dass eine maximale Genauigkeit der Bestimmung der zugeordneten ausgewählten Merkmale von Objekten erreicht wird, wobei zugleich zumindest die Stabilität der Klassifizierung erhalten bleiben soll. Es soll somit eine Anpassung zwischen bestmöglicher Klassifizierung und hoher Robustheit der Klassifizierung angestrebt werden. Gleichzeitig soll ein Übertrainieren des Klassifikators vermieden werden.

Die Aufgabe wird mit den Merkmalen des Patentanspruchs 1 gelöst.

In dem Verfahren zur Klassifizierung von Spektren von Objekten mit komplexem Informationsgehalt mit mindestens zwei unterschiedlichen Objektinformationen, unter Einsatz eines Verfahrens zur Registrierung und zur Vorbehandlung von spektralen Daten und eines der Datenvorbehandlung zugehörigen Verfahrens der Klassifizierung mit der Berechnung eines Klassifikators, wird gemäß dem Kennzeichenteil des Patentanspruchs 1 nach der Registrierung der Spektren und der Vorbehandlung von spektralen Daten ein multiples Klassifizierungsverfahren mit mindestens zwei unterschiedlichen Verfahren der Datenvorbehandlung spektraler Daten und eines der jeweiligen Datenvorbehandlung zugeordneten Verfahrens zur Klassifizierung durchgeführt.

Im Rahmen der Erfindung soll unter der Registrierung von von Spektren die Aufnahme, Ermittlung und Speicherung von Spektren und die Erzeugung von digitalisierten Signalen zur Speicherung verstanden werden, die für die weitere Datenvorbehandlung der spektralen Daten zur Verfügung stehen.

In den Datenvorbehandlungen werden je nach eingesetztem Vorbehandlungs-Algorithmus unterschiedliche korrigierte, vorbehandelte Spektren mit vielen Datenpunkten erzeugt, die mindestens einem Verfahren zur Klassifizierung zugeordnet werden.

Dabei werden folgende Schritte nach Registrierung und Datenvorbehandlung in dem Auswerteverfahren von registrierten Spektren von Objekten durchgeführt:

- eine Berechnung mehrerer Klassifikatoren pro Art der Datenvorbehandlung,
- Berechnen mehrerer Klassifikatoren pro Art der Datenvorbehandlung durch iterative Berechnung und Validierung ausgehend vom Trainingsset,
- Klassifizieren der vorbehandelten Spektren des Testsets mit allen ausgehenden vom Trainingsdatenset berechneten Klassifikatoren,
- - Einordnen der Spektren in eine Klasse von Objektinformationen mit einem Ausdruck einer Wahrscheinlichkeit zur Klassifikationszugehörigkeit,
- Berechnen eines Klassifikationsergebnisses durch Berechnen eines Medians oder durch Durchführen einer Clusteranalyse zur Darstellung des Wahrscheinlichkeitsergebnisses der einer Klasse zugehörigen Objektinformationen.

Bei der Festlegung und Ermittlung der Anzahl der berechneten Klassifikatoren $N_G$ in den Serien je Klassifizierungsgruppe werden sowohl der Umfang der spektralen Datenpunkte $v_S$ und die doppelte Halbbreite der spektralen Regionen $w_S$ als auch die Anzahl der ausgewählten spektralen Regionen $R_S$ für die Klassifizierung in Gleichung (I) berücksichtigt:

$$N_G = \frac{v_S}{2w_S \cdot R_S} \qquad\qquad (I)$$

wobei mit der Gleichung (I) sichergestellt wird, dass jeder Datenpunkt $v_S$ mit gleicher Wahrscheinlichkeit ausgewählt werden kann.

Die zu einem Umfang der spektralen Datenpunkte gehörenden Datenpunkte können aber auch gewichtet werden.

Zumindest eine der Spektrenvorbehandlungen wird derart strukturiert, dass jeweils bestimmte Merkmale hervortreten und andere bestimmte Merkmale unterdrückt werden, so dass unterschiedlich bestimmte Merkmale für die Klassifikation eingesetzt werden.

Zumindest eine Spektrenvorbehandlung kann mit gleich bestimmten Merkmalen ausgebildet sein und zumindest einer der vorgenannten Spektrenvorbehandlungen mit unterschiedlich bestimmten Merkmalen für die Klassifikation eingesetzt werden.

Die vorbehandelten Spektren können als variable Trainingssets ausgelegt werden, und mehrere Klassifikatoren der Serien bzw. Klassifikatoren werden iterativ bestimmt und validiert.

Im Rahmen der Erfindung soll unter der Klassifizierung die nach einem vorgegebenen Algorithmus bestimmte Einordnung der vorbehandelten Spektren in eine jeweilige Klasse verstanden werden. Dabei wird das Verfahren der Klassifizierung mit Hilfe vorgegebener Parameter durchgeführt und das Ergebnis der Klassifikation durch einen berechneten Klassifikator ausgedrückt.

Es kann mindestens ein Verfahren der gestützten Klassifizierung und/oder der nichtgestützten Klassifizierung zur Selektion spektraler Regionen oder einzelner Wellenlängenbereiche und nachfolgender Analyse eingesetzt werden. Dabei kann eine lineare oder nichtlineare Diskriminanzanalyse eingesetzt werden.

Es können zur Klassifizierung auch Verfahren der neuronalen Netze und/oder ein Verfahren der linearen Zeit-Frequenz-Transformationen (engl. Wavelets) eingesetzt werden.

Es können dabei die Spektren der optischen Molekülspektroskopie, wie Absorption, Emission, Streuung oder UV/vis, NIR, IR Absorption, Fluoreszenz oder Raman, klassifiziert werden.

Als Datenvorbehandlungen der registrierten spektralen Daten oder Rohspektren können Basislinienkorrekturen, Normierungen, Ableitungen, Covarianz und/oder eine Hauptkomponentenanalyse eingesetzt werden.

Zur Bewertung der Klassifikatoren für ein Klassifikationsergebnis kann eine Berechnung eines Medians oder eine Durchführung einer Clusteranalyse vorgesehen werden.

Dabei wird der Median oder Zentralwert als ein Mittelwert für Verteilungen in der Statistik angegeben. Der Median einer Auflistung von Zahlenwerten ist der Wert, der an der mittleren (zentralen) Stelle steht, wenn die Werte der Größe nach sortiert werden. Der Wert der Größe stellt hier jeweils den Punktwert eines Klassifikators oder die durch den Klassifikator ermittelte Wahrscheinlichkeit der Klassenzugehörigkeit dar.

Das erfindungsgemäße Verfahren wird mit folgenden Schritten im Detail absolviert:

- Aufnahme und Registrierung der Spektren mittels zumindest einer optischen Einrichtung mit zumindest einem Spektrometer und/oder weiteren Detektoren,
- Erzeugung von digitalisierten Signalen in Form von Datenpunkten und Speicherung der registrierten Spektren in Speichereinheiten von Klassifikationseinheiten einer Auswerteeinheit,
- Durchführen einer Spektrenvorbehandlung, indem die registrierten und gespeicherten Spektren in den einzelnen Speichereinheiten einzeln vorbehandelt werden und die zugehörigen digitalisierten ausgewerteten Signale zur Weiterverarbeitung bereitgestellt werden,
- Trennung der vorbehandelten Spektren als Trainingsset und als Testset,
- Auslegung und Einsetzung der vorbehandelten Spektren als Trainingsset und von einem vom Trainingsset getrennten Testset,
- Berechnen mehrerer Klassifikatoren pro Art der Datenvorbehandlung durch iterative Berechnung und Validierung ausgehend vom Trainingsset, indem spektrale Regionen $R_S$ aus einer Koordinate von relativen Wellenzahlen ausgewählt werden und die Intensitätswerte der ausgewählten Regionen $R_S$ nachfolgend mittels Diskriminanzanalyse klassifiziert werden, wobei in einem wiederholten Schritt eine erneute Auswahl spektraler Regionen $R_S$ und die Klassifizierung der Intensitätswerte erfolgt, und wobei der Zyklus iterativ bis zum Erreichen einer nicht mehr verbesserbaren Genauigkeit unter Vorgabe eines Abbruchkriteriums wiederholt wird,
- Klassifizieren der vorbehandelten Spektren des Testsets mit allen ausgehenden vom Trainingsdatenset berechneten Klassifikatoren,
- - Einordnen der Spektren in eine Klasse von Objektinformationen mit einem Ausdruck einer Wahrscheinlichkeit zur Klassifikationszugehörigkeit,
- Berechnung eines Klassifikationsergebnisses durch Berechnung des Medians oder durch Durchführung einer Clusteranalyse zur Darstellung des Wahrscheinlichkeitsergebnisses der einer Klasse zugehörigen Objektinformationen.,

Als Objekte mit Objektinformationen können jegliche Vogeleier, wahlweise Hühnereier eingesetzt werden und als Objektinformationen können in einem speziellen Anwendungsfall die dualen Informationen über das weibliche Eigeschlecht oder über das männliche Eigeschlecht benutzt werden.

Das Verfahren enthält somit Schritte zur Durchführung einer multiplen Klassifikation, beruhend auf unterschiedlichen herkömmlichen Auswerteverfahren nach einer der Spektrendetektierung und -registrierung nachgeordneten Spektrenvorbehandlung und einer nachfolgenden mehrfachen Berechnung von unterschiedlichen Klassifikatoren. Dabei ist zumindest eine Spektrenvorbehandlung beteiligt, bei der die Spektrenvorbehandlung so strukturiert ist, dass bei Berücksichtigung der Gleichwertigkeit von Merkmalen auch eine Berücksichtigung jeweils bestimmter Merkmale deutlicher hervortritt und andere Merkmale stärker unterdrückt werden. Die so vorbehandelten Spektren werden nun als Trainingsset ausgelegt, wobei mehrere Serien von Klassifikatoren berechnet werden. Die Klassifikatoren werden iterativ berechnet und validiert. Auf diese Art lassen sich mehrere Klassifikatoren bestimmen. Die Spektren des Testsets werden nachfolgend mit allen Klassifikatoren klassifiziert. Die Einordnung der Spektren in eine bestimmte Klasse der Objektinformationen/Merkmale (bei Hühnern: männlich, weiblich) geschieht dabei vorzugsweise als Punkt-Wert (engl. Score) oder in einem Ausdruck einer Wahrscheinlichkeit zur Klassenzugehörigkeit. Um aus den Klassifikatoren eine alleinige Aussage zu gewinnen, werden die Verhältnisse innerhalb der Klassifikatoren ermittelt. Ein einfaches Mittel zur Verhältnisdarstellung ist hierzu z.B. eine Berechnung des Medians oder eine Clusteranalyse.

Eine Vorrichtung zur Klassifizierung von Spektren von Objekten mit komplexem Informationsgehalt, vorzugsweise mit Objekten in Form von Hühnereiern für eine Bestimmung einer dualen Eiinformation - weiblich oder männlich -, wobei in der Vorrichtung das vorgenannte Verfahren realisiert wird, kann zumindest folgende Einheiten umfassen

- zumindest eine detektierende optische Einrichtung mit zumindest einem Spektrometer und/oder weiteren Detektoren zur Aufnahme und Registrierung der Spektren,
- eine Einheit zur Erzeugung von digitalisierten Signalen in Form von Datenpunkten, die die Spektren realisieren.
- Speichereinheiten zur Speicherung der registrierten Spektren in den Klassifikationseinheiten/-gruppen einer die Klassifikationseinheiten umfassenden Auswerteeinheit,

- Einheiten zur Spektrenvorbehandlung, in denen die registrierten Spektren einzeln vorbehandelt werden und die zugehörigen digitalisierten ausgewerteten Signale - die ermittelten Datenpunkte - zur Weiterverarbeitung bereitgestellt werden,
- Trainingssets zur Auslegung und Einsetzung der vorbehandelten Spektren,
- Mindestens eine Klassifizierungseinheit zur Berechnung mehrerer Klassifikatoren pro Art der Datenvorbehandlung iterativen Verfahren und einer Validierung ausgehend vom Trainingsset in den Klassifizierungseinheiten,
- Testsets zur Klassifizierung der vorbehandelten Spektren mit allen Klassifikatoren pro Art der Datenvorbehandlung,
- eine Einheit zur Einordnung der vorbehandelten Spektren in mindestens eine duale Klasse - bei Hühnern: männlich oder weiblich - von Objekt(Ei)informationen mit einem Ausdruck einer Wahrscheinlichkeit zur Klassenzugehörigkeit,
- eine Bewertungseinheit zur Berechnung des Klassifikationsergebnisses z.B. in Form des Medians oder mittels Durchführung einer Clusteranalyse zur Bestimmung des Wahrscheinlichkeitsergebnisses zumindest einer der dualen Klasse - bei Hühnern: weiblich oder männlich - zugehörigen Objekt(Ei)information.

Weiterbildungen und weitere Ausgestaltungen der Erfindung sind in weiteren Unteransprüchen angegeben.

Die Erfindung wird mittels Ausführungsbeispielen anhand von Zeichnungen erläutert.

Es zeigen:

Fig. 1    eine schematische Block-Darstellung eines erfindungsgemäßen Verfahrens zur Klassifizierung von Spektren von Objekten mit komplexem Informationsgehalt, insbesondere von optischen Molekülspektren zur Zuordnung und Bestimmung von dualen Objektinformationen, wobei das Verfahren als multiples Klassifizierungsverfahren ausgeführt ist,

Fig. 2a   eine schematische Darstellung eines einzelnen Klassifizierungsverfahrens mit Spektrenvorbehandlung: Rohspektren, wobei alle Merkmale als gleichwertig berücksichtigt werden (gleich große Kreise),

Fig. 2b   eine schematische Darstellung eines einzelnen Klassifizierungsverfahrens mit Spektrenvorbehandlung: Linearer Basislinienkorrektur, mit einem favorisierten großen Merkmalskreis der Fluoreszenzintensität und mehreren weniger relevanten kleinen Merkmalskreisen,

Fig. 2c   eine schematische Darstellung eines einzelnen Klassifizierungsverfahrens mit Spektrenvorbehandlung: Normierung, mit einem favorisierten großen Merkmalskreis des Fluoreszenzprofils und mehreren weniger relevanten kleinen Merkmalskreisen,

Fig. 2d   eine schematische Darstellung eines einzelnen Klassifizierungsverfahrens mit Spektrenvorbehandlung: Ramanspektren, mit einem favorisierten großen Merkmalskreis der molekularen Zusammensetzung und mehreren weniger relevanten kleinen Merkmalskreisen,

Fig. 3a   eine schematische Darstellung der dem Rohspektrum-Klassifizierungsverfahren zugeordneten Spektren gemäß Fig. 2a, wobei das punktierte Spektrum dem weiblichen Hühnerei-Spektrum zugeordnet ist,

Fig. 3b   eine schematische Darstellung der dem linearen Basislinienkorrektur- Klassifizierungsverfahren zugeordneten Spektren gemäß Fig. 2b, wobei das punktierte Spektrum dem weiblichen Hühnerei-Spektrum zugeordnet ist,

Fig. 3c   eine schematische Darstellung der dem Nomierungs-Klassifizierungsverfahren zugeordneten Spektren gemäß Fig. 2c, wobei das punktierte Spektrum dem weiblichen Hühnerei-Spektrum zugeordnet ist,

Fig. 3d   eine schematische Darstellung der dem Ramanspektrum-Klassifizierungsverfahren zugeordneten Spektren gemäß Fig. 2d, wobei das punktierte Spektrum dem weiblichen Hühnerei-Spektrum zugeordnet ist,

Fig. 4a   eine schematische Darstellung des Ablaufs einer Klassifizierung mit Ermittlung eines Klassifikators nach dem Stand der Technik,

Fig. 4b   ein Flussdiagramm für das erfindungsgemäße multiple Klassifizierungsverfahren mit Trainingsset und Testset in algorithmischer Verbindung mit Klassifikationsergebnis-Gestaltung aus einer großen Anzahl von Klassifikatoren,

Fig. 5    eine schematische Darstellung einer Wahrscheinlichkeits/Klassifikatoranzahl-Säulendarstellung für zwanzig Klassifikatoren gemäß Fig. 1 für ein als Beispiel verwendetes Hühner-Ei, wobei die über der gestrichelten Linienführung - Trenngrenze - liegenden Säulen dem weiblichen Geschlecht zugeordnet werden,

Fig. 6    eine Draufsicht auf die Säulendarstellung eines Eies gemäß der Fig. 5 und eine mögliche Ansicht auf einem Display,

Fig. 7    eine Wahrscheinlichkeits(weiblich)/Klassifikatorenanzahl-Darstellung mit der Angabe des berechneten Medians für ein Ei mit den 20 Klassifikatoren gemäß Fig. 6, wobei eine fettgestrichelte Trenngrenze bei 0,5 der Wahrscheinlichkeit und ein dünngestrichelter Medianwert bei etwa 0,72 der Wahrscheinlichkeit "weiblich" liegen, so dass das Geschlecht des Eies als weiblich identifiziert werden kann,

Fig. 8    eine schematische Darstellung einer Wahrscheinlichkeits/Klassifikatoranzahl-Säulendarstellung für ein Ei für wahlweise 120 Klassifikatoren, wobei die über der fettgestrichelten Linienführung - Trenngrenze - liegenden Endbereiche der Säulen dem weiblichen Geschlecht eines Eies und die unter der fettgestrichelten Trenngrenze liegenden Endbereiche der Säulen dem männlichen Geschlecht eines Eies zugeordnet werden,

Fig. 9    eine Darstellung des berechneten, gestrichelt dargestellten Medians für ein als weiblich erkanntes Ei mit den 120 Klassifikatoren gemäß Fig. 8 in einer Wahrscheinlichkeits/Klassifikatoranzahl-Darstellung,

Fig. 10a    eine schematische Darstellung eines einzelnen Klassifizierungsverfahrens mit Spektrenvorbehandlung: Rohspektren, wobei alle Merkmale als gleichwertig berücksichtigt werden, mit acht ausgewählten spektralen Regionen $R_{S1}$ bis $R_{S8}$ bei einem Umfang der spektralen Datenpunkte im gesamten Spektralbereich zwischen den Wellenzahlen 570 cm$^{-1}$ bis 2750 cm$^{-1}$ zur Ermittlung der Anzahl der Klassifikatoren pro Datenvorbehandlung,

Fig. 10b    einen vergrößerten Ausschnitt (I-I) der Datenpunktdarstellung in der vorgebenen spektralen Region $R_{S8}$ gemäß Fig. 10a,

Fig. 10c    einen vergrößerten Ausschnitt (I-I) der Datenpunktdarstellung in der spektralen Region $R_{S8}$ mit einer Angabe der Wichtung von Datenpunkten im Bereich der Region $R_{S8}$ gemäß Fig. 10a und Fig. 10b,

Fig. 11a    eine Darstellung des berechneten, gestrichelt dargestellten Medians für ein als männlich erkanntes Ei mit den 120 Klassifikatoren ähnlich der Fig. 8 in einer Wahrscheinlichkeits/Klassifikatoranzahl-Darstellung,

Fig. 11b    eine erste Histogramm-Darstellung der Abhängigkeit zwischen der Anzahl von Elementen des Clusters und dem Schwerpunkt des Clusters und

Fig. 11c    eine zweite Histogramm-Darstellung der Abhängigkeit zwischen der Anzahl von Elementen des Clusters und dem Schwerpunkt des Clusters.

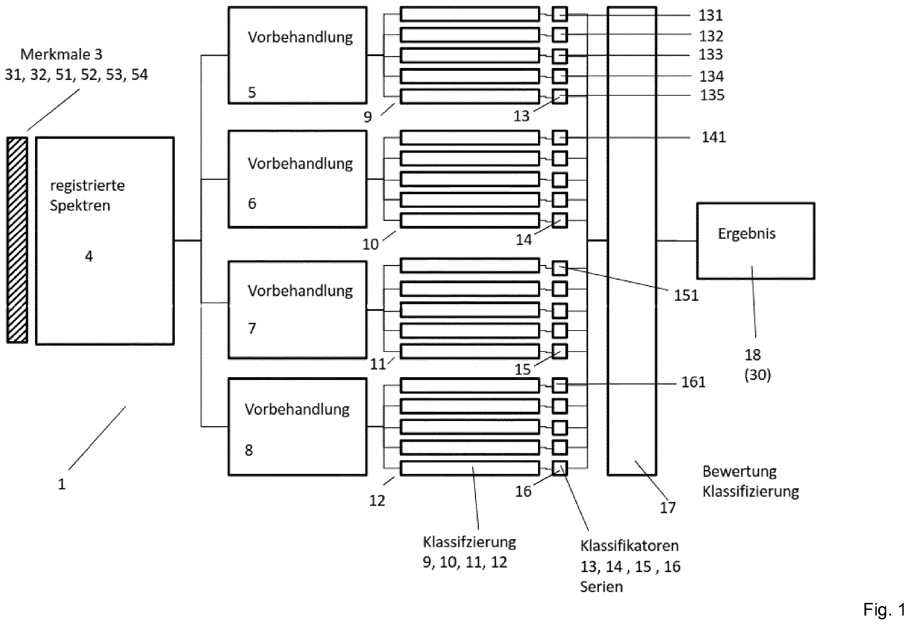

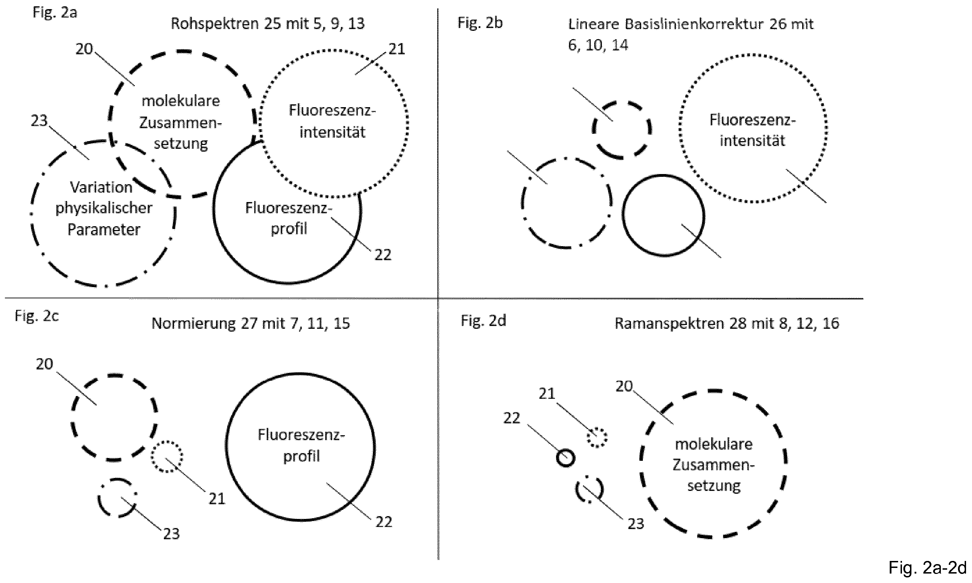

Im Folgenden werden die Fig. 1 und die Fig. 2a, 2b, 2c, 2d gemeinsam betrachtet. In Fig. 1 ist in einer schematischen Block-Darstellung ein erfindungsgemäßes Verfahren 1 zur Klassifizierung von Spektren 4 eines Objektes 2 mit komplexem Informationsgehalt mit mindestens zwei/dualen und unterschiedlichen Objektinformationen/Merkmalen, insbesondere von optischen Molekülspektren 4 zur Zuordnung von Objektinformationen/Merkmale 3 für eine wahrscheinliche Bestimmung einer beispielsweisen dualen Objektinformation 31, 32 gezeigt.

Als zu untersuchende Objekte 2 können Vogeleier, beispielsweise Hühnereier eingesetzt werden und als duale Objektinformationen 3 können beispielsweise das Merkmal 31 über das weibliche Eigeschlecht und das Merkmal 32 über das männliche Eigeschlecht gesucht und bestimmt werden.

Es folgt die Beschreibung des erfindungsgemäßen Verfahrens 1 zur Durchführung der Klassifizierung.

Dazu ist in der Fig. 1 eine blockweise Abfolge des erfindungsgemäßen Verfahrens 1 gezeigt.

In dem Verfahren 1 zur Klassifizierung von Spektren 4 von Objekten 2 mit komplexem Informationsgehalt mit mindestens zwei unterschiedlichen Objektinformationen erfolgt nach der Registrierung unter Einsatz eines Verfahrens der Vorbehandlung von Daten und eines der Datenvorbehandlung zugehörigen Verfahrens der Klassifizierung die Berechnung eines Klassifikators.

Erfindungsgemäß wird nach Registrierung und Datenvorbehandlung von Spektren 4 ein multiples Klassifizierungsverfahren mit mindestens zwei unterschiedlichen Verfahren der Datenvorbehandlung 5, 6, 7, 8 der Spektren 4 und des der jeweiligen Datenvorbehandlung 5, 6, 7, 8 zugeordneten Verfahrens zur Klassifizierung in den Gruppen 9, 10, 11, 12 zur Ermittlung von mehreren, z.B. fünf Klassifikatoren je Gruppe 9, 10, 11, 12, also von insgesamt vielen, z.B. zwanzig (fünf Klassifikator/Gruppe x vier Guppen) Klassifikatoren 131, 132, 133, 134, 135, usw. für die Serien 14, 15, 16 durchgeführt.

Dabei werden folgende Schritte nach Registrierung und Datenvorbehandlung der Spektren durchgeführt, wobei sich die Schritte auf die Fig. 1 beziehen:

- eine Berechnung von fünf Klassifikatoren der Serien 13, 14, 15, 16 pro Art der Datenvorbehandlung 5, 6, 7, 8, so dass schließlich zwanzig Klassifikatoren 131, 132, 133, 134, 135, usw. ermittelt werden,
- eine Ermittlung der fünf Klassifikatoren der Serien 13, 14, 15, 16, iterativ angepasst und validiert,
- eine Berechnung von Wahrscheinlichkeiten der Klassenzugehörigkeit,
- eine gleichberechtigte Einziehung aller fünf Klassifikatoren der Serien 13, 14, 15, 16 bzw. Klassifikatoren 131, 132, 133, 134, 135 usw. in die Ermittlung eines Klassifikationsergebnisses 18, z.B. in Form eines Medians 30.

Bei der Festlegung bzw. Ermittlung der Anzahl der zu berechnenden Klassifikatoren $N_G$ in den Serien 13, 14, 15, 16 bezogen auf die Gruppen 9, 10, 11, 12 werden ein Umfang der spektralen Datenpunkte $v_S$ und eine doppelte Halbbreite $w_S$ von spektralen Regionen $R_S$ als auch eine Anzahl der ausgewählten spektralen Regionen $R_S$ in folgender Gleichung (I) berücksichtigt:

$$N_G = \frac{v_S}{2w_S \cdot R_S} \tag{I}$$

wobei mit der Gleichung (I) sichergestellt wird, dass jeder Datenpunkt $v_S$ mit gleicher Wahrscheinlichkeit ausgewählt werden kann.

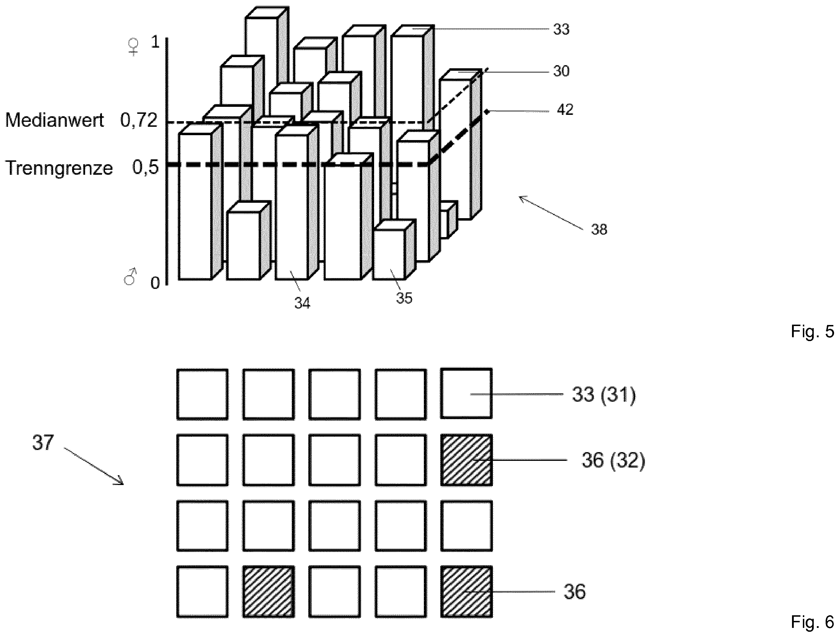

Für insgesamt zwanzig Klassifikatoren der vier Serien 13, 14, 15, 16 mit $N_G$ (13), $N_G$ (14), $N_G$ (15) und $N_G$ (16) gemäß Fig. 1 sowie gemäß der Fig. 5, Fig. 6 und Fig. 7 sind für den gesamten Spektralbereich von 500 cm$^{-1}$ bis 2750 cm$^{-1}$ beispielsweise folgende Parameter vorgegeben:

Umfang der spektralen Datenpunkte $v_S$ in einem vorgegebenen gesamten Spektralbereich 500 cm$^{-1}$ bis 2750 cm$^{-1}$ mit $v_S$ = 800,
Anzahl der ausgewählten spektralen Regionen $R_S$ mit $R_S$ = 8,
Breite B der spektralen Regionen $R_S$ mit B = 2 · $w_S$ = 5, d.h. in einer Region $R_S$ können sich zwanzig Datenpunkte $v_S$ befinden. Die Halbbreite $w_S$ beträgt damit $w_S$ = 2,5.

Die Spektrenvorbehandlungen 6, 7, 8 können gemäß Fig. 2b, 2c, 2d derart strukturiert werden, dass jeweils bestimmte Merkmale favorisiert werden und hervortreten und andere Merkmale unterdrückt werden.

Bei der Spektrenvorbehandlung 5 der Rohspektren 25 gemäß Fig. 2a können alle berücksichtigten Merkmale als gleichberechtigt behandelt und vorbehandelt werden.

Die vorbehandelten Spektren 4 werden als Trainingsset 24 gemäß Fig. 4b ausgelegt und mehrere Klassifikatoren der Serien 13, 14, 15, 16 bzw. z.B. im Detail für Serie 13 die Klassifikatoren 131, 132, 133, 134, 135 usw. iterativ bestimmt und validiert.

Nach Durchlauf mindestens zweier mit je einer vorangegangenen spektrumunterschiedlichen Datenvorbehandlung 5, 6, 7, 8 versehenen Klassiizierungsverfahren 9, 10, 11, 12 mit mindestens einem ermittelten Klassifikator 131, 141, 151, 161 können gemäß Fig. 1 gesamtheitlich mindestens zwei der ermittelten Klassifikatoren 131, 141, 151, 161 zur Bewertung und der nachfolgenden Ermittlung eines Wahrscheinlichkeitsergebnisses 18 bezüglich der vorgegebenen unterschiedlichen Objektinformationen 31, 32 erreicht und eingesetzt werden, wobei das Wahrscheinlichkeitsergebnis 18 ausgegeben wird, so dass ein Schluss zumindest auf die mit einem höchsten Wert ermittelte Objektinformation 31 oder 32 ermöglicht wird.

Es kann mindestens ein Verfahren der gestützten Klassifizierung und/oder der nichtgestützten Klassifizierung zur Selektion spektraler Regionen $R_S$ oder einzelner Wellenlängenbereiche/Wellenzahlbereiche und nachfolgender Analyse eingesetzt werden.

Die nachfolgende Analyse kann eine lineare Diskriminanzanalyse oder eine nichtlineare Diskriminanzanalyse sein.

Es kann aber auch als ein Verfahren zur Klassifizierung in den Gruppen 9, 10, 11, 12 ein Verfahren der neuronalen Netze und/oder ein Verfahren der linearen Zeit-Frequenz-Transformationen (engl. Wavelets) eingesetzt werden.

Die Spektren 4 der optischen Molekülspektroskopie, wie Absorption, Emission, Streuung bzw. UV/vis, NIR, IR Absorption, Fluoreszenz, Raman können mit dem erfindungsgemäßen Verfahren klassifiziert werden.

Zu den in Fig. 1 gezeigten Datenvorbehandlungen 5, 6, 7, 8 können Rohspektren 25, Basislinienkorrekturen 26, Normierungen 27, Ableitungen, Covarianz und/oder eine Hauptkomponentenanalyse/Ramanspektren 28 festgelegt und eingesetzt werden. Eine Datenvorbehandlung besteht in der Ausbildung von digitalen Signalen, also von Datenpunkten, die aneinandergereiht die jeweilige berechnete Spektralkurve von 25, 26, 27, 28 ergeben und die somit den einzelnen eingesetzten unterschiedlichen Klassifizierungsverfahren zugeordnet werden können.

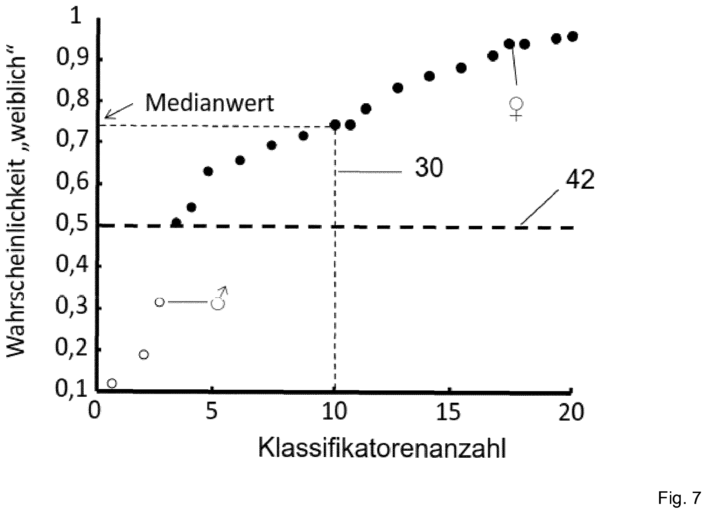

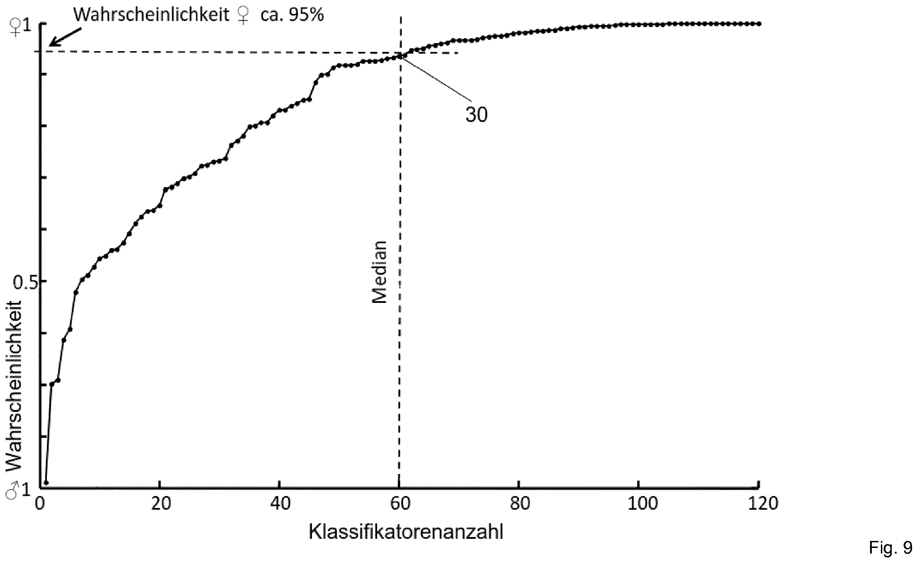

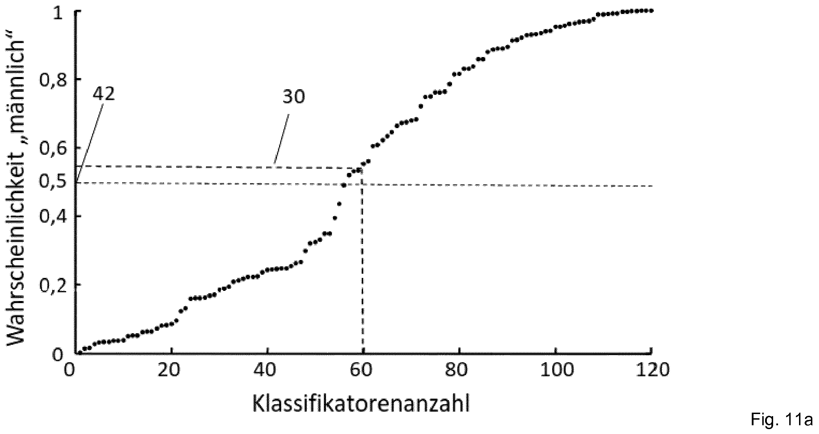

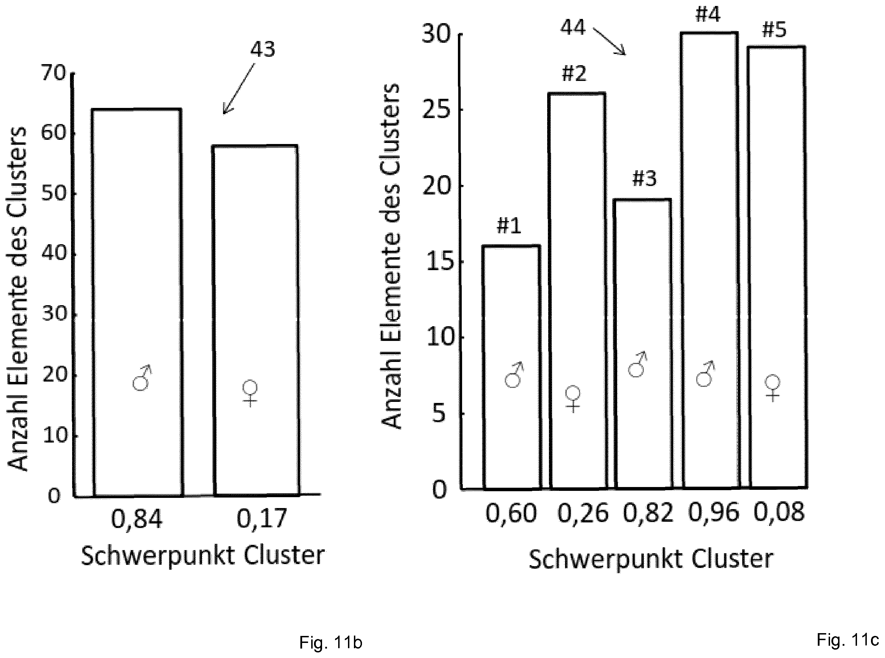

Zur Bewertung der Klassifikatoren der Serien 13, 14, 15, 16 für ein Klassifikationsergebnis 18 kann eine Berechnung eines Medians 30 (Fig. 7. Fig. 9, Fig. 11a) für ein Objekt 2 oder eine Durchführung (Fig. 11a, Fig. 11b) einer Clusteranalyse vorgesehen werden.

Als Beispiel für eine Auswertung kann die bekannte k-means Clusteranalyse eingesetzt werden. Dabei werden in Fig. 11a mindestens zwei Cluster für "männlich" bzw. "weiblich" vorgegeben. Jener Cluster, dem die meisten Elemente, d.h. Wahrscheinlichkeiten, zugeordnet werden, definiert das Geschlecht (Fig. 11b - männlich). Als Cluster wird eine Gruppe von zusammengestellten Elementen mit ähnlichen Eigenschaften (hier: Wahrscheinlichkeiten - Klassifikationsergebnisse) bezeichnet.

Die Fig. 11a zeigt eine Kurve der Abhängigkeit zwischen Wahrscheinlichkeit mit einem Merkmal "männlich" und einer Klassifikatorenanzahl von 120 Klassifikatoren. Im Plot der sortierten Wahrscheinlichkeiten zeigt sich, dass der Median 30 knapp oberhalb der Trenngrenze 42 von 0,5 der Wahrscheinlichkeitskoordinate liegt. Damit wird das Ei 2 gerade noch als männlich klassifiziert werden. Die bekannte k-means Clusteranalyse führt hier zu einem deutlicheren Ergebnis.

Dazu sind in der Fig. 11b eine erste Histogramm-Darstellung 43 der Abhängigkeit zwischen der Anzahl von Elementen des Clusters und dem Schwerpunkt des Clusters und in der Fig. 11c eine zweite Histogramm-Darstellung 44 der Abhängigkeit zwischen der Anzahl von Elementen des Clusters und dem Schwerpunkt des Clusters gezeigt, wobei als Elemente die erreichten Klassifikationsergebnisse gelten.

Dazu sind in Fig. 11b zwei Cluster gebildet, deren Schwerpunkte bei 0,84 und 0,17 liegen. Da dem Cluster mit dem Schwerpunkt 0,84 mehr Elemente, d.h. Klassifikationsergebnisse, zugeordnet werden, kann das Ei 2 als eindeutig männlich bewertet werden.

Bei der Wahl von fünf Clustern in der Histogramm-Darstellung 44 gemäß Fig. 11c wird das Ergebnis ebenso bestätigt. Hier werden 65 berechnete Wahrscheinlichkeitswerte als männlich eingeordnet, wobei der Cluster (Nummer 4 = #4) mit dem stärksten Wert 0,96 für "männlich" auch die meisten Elemente gemäß Fig. 11a enthält. Damit ist das Geschlecht des Eies 2 eindeutig "männlich".

Gleiches und Ähnliches gilt auch für die Clusteranalyse, wenn das Geschlecht des Eies 2 als weiblich ermittelt wird.

Das erfindungsgemäße Verfahren 1 kann mittels folgender Schritte unter Verwendung von Hardware-Komponenten einer zugehörigen Vorrichtung realisiert werden:

- Aufnahme und Registrierung der Spektren 4 mittels zumindest einer optischen Einrichtung mit zumindest einem Spektrometer und/oder weiteren Detektoren,
- Erzeugung von digitalisierten Signalen, den Datenpunkten, und Speicherung der detektierten Spektren 4 in Speichereinheiten der Klassifikationseinheiten einer Auswerteeinheit,
- Spektrenvorbehandlung 5, 6, 7, 8, indem die aus den Datenpunkten $v_S$ bestehenden gespeicherten Spektren 4 in den einzelnen Speichereinheiten einzeln ausgewertet werden und die zugehörigen digitalisierten ausgewerteten Signale zur Weiterverarbeitung bereitgestellt werden,
- Auslegung oder Ausbildung der vorbehandelten Spektren 25, 26, 27, 28 als Trainingsset 19 und als davon getrenntes Testset 29,
- Berechnung der Klassifikatoren der Serien 13, 14, 15, 16 in Form von einzelnen Klassifikatoren 131, 132, 133, 134, 135 einer Serie 13 usw. der berücksichtigten einzelnen Klassifizierungsverfahren 9, 10, 11, 12 unter Einbeziehung von iterativen Verfahren und einer Validierung in den Klassifizierungseinheiten/-gruppen,
- Klassifizierung der ausgewerteten Spektren 25, 26, 27, 28 des Testsets 24 mit allen Klassifikatoren der Serien 13, 14, 15, 16,
- Einordnung der Spektren 25, 26, 27, 28 in eine Klasse von Objektinformationen mit einem Ausdruck einer Wahrscheinlichkeit zur Klassenzugehörigkeit,
- Berechnung des Medians 30 oder Durchführung einer vordem genannten Clusteranalyse zur Darstellung des Wahrscheinlichkeitsergebnisses in Form eines Klassifikationsergebnisses 18 der einer Klasse zugehörigen Objektinformationen.

Der Aufbau von Klassifikatoren bezüglich der Serien 13, 14, 15, 16 mittels eines Trainingssets 19 wird durch ein Testset 29 mit z.B. maximal 30% der Spektren (gestrichelte Linie zu den Klassifikatoren 13, 14, 15, 16) gemäß Fig. 4b verifiziert, so dass im Ergebnis ein klassifiziertes Testset 24 (gestrichelte Linie von den Klassifikatoren der Serien 13,14, 15, 16 zum klassifizierten Testset 24) erreicht wird.

Dazu ist zu bemerken, dass im Allgemeinen die registrierten in-ovo Spektren 4 naturgemäß hochvariabel sind. Dies begründet sich zum einen in der immanenten Variabilität biologischer Systeme und zum anderen in der Sensitivität der ramanspektroskopischen Messungen.

Externe Störungen systematischer und zufälliger Art führen zu einer hohen Variabilität der spektralen Merkmale und überlagern somit die geschlechtsrelevanten Informationen.

Weiterhin tritt bei dem Verfahren der Ramanspektroskopie auch Fluoreszenzlicht auf, das zwar ebenso molekulare Informationen enthält, jedoch gleichzeitig die in der Regel viel schwächeren Ramanspektroskopischen molekularen Informationen über die Zusammensetzung des untersuchten Objektes 2 überlagert.

In Fig. 2a ist eine schematische Darstellung der Rohspektren 25 als eines aller berücksichtigten einzelnen Klassifizierungsverfahren in Fig. 1 der vier einzelnen Klassifizierungsverfahren angegeben.

Generell lassen sich gemäß Fig. 2a, Fig. 2b, Fig, 2c und Fig. 2d mindestens vier Klassen von Signalen bzw. bestimmten Merkmalen bilden:

- molekulare Zusammensetzung 20,
- Fluoreszenzintensität 21,
- Fluoreszenzprofil 22 und
- Variation physikalischer Parameter 23,

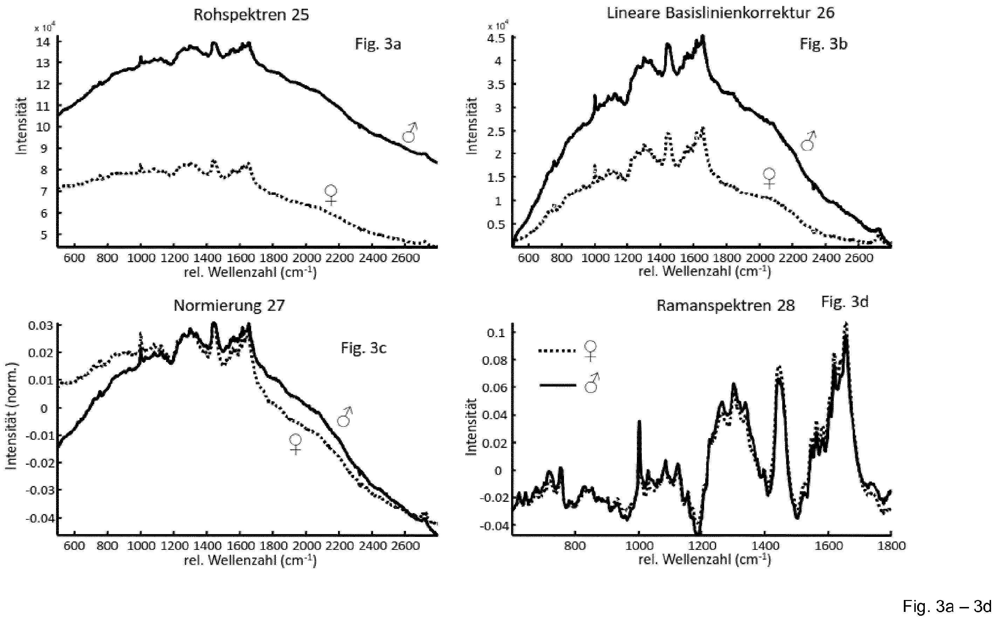

wobei diese bestimmten Merkmale 20, 21, 22, 23 zur sichtbaren Darstellung in Fig. 3a, Fig. 3b, Fig. 3c und Fig. 3d als gleich große umrandete und/oder unterschiedlich gestrichelt umrandete Kreise ausgebildet sind.

Hinter den Klassifikatoren steht jeweils ein mathematischer Ausdruck zur Trennung der Signale gemäß der Objektinformationen 3 (31 weiblich, 32 männlich).

Drei Klassen/Merkmale 20, 21, 22 der vier Klassen/Merkmale 20, 21, 22, 23 enthalten geschlechtsrelevante Informationen. Allerdings ist es nicht möglich, die Variation 23 der physikalischen Parameter aus den Spektren 4 so zu eliminieren, dass kein oder nur ein geringer Informationsverlust in den drei anderen Klassen 20, 21, 22 entsteht. Die Rohspektren 25 haben somit durch die Gleichberechtigung aller bestimmten Merkmale den höchsten Gehalt an allen Informationen, aber auch den höchsten Gehalt an Störungen. Durch Hinzunahme mindestens einer der genannten Datenvorbehandlung z.B. 26 aus den Datenvorbehandlungen 26, 27, 28 mit unterschiedlich bewerteten Merkmalen werden die Störungen verringert. Durch Einsatz weiterer Datenvorbehandlungen 27, 28 werden die ursprünglichen Störungen minimiert oder sogar beseitigt.

In Fig. 2b ist gezeigt, dass die als digitale Signale registrierten in-ovo Spektren 4 einer linearen Basislinienkorrektur 26 unterzogen werden, wobei sich das Fluoreszenzintensitätssignal 21 (großer Kreis) heraushebt. Gleichzeitig werden Signale 23 physikalischer Parameter in den Spektren unterdrückt (kleiner Kreis). Aufgrund der üblicherweise großen Intensitätsunterschiede zwischen Fluoreszenzsignal und Ramansignal treten auch die Informationen zur molekularen Zusammensetzung 20 (kleiner Kreis) in den Hintergrund. Die Fluoreszenzintensität 21 (großer Kreis) selbst ist jedoch ein potenzieller Marker für die Geschlechtserkennung, da männliche Embryonen häufig, jedoch nicht immer, eine biochemische Komposition im Blut aufweisen, die gegenüber weiblichen Embryonen bzw. dem Blut weiblicher Embryonen eine erhöhte Fluoreszenzintensität 21 aufweisen.

In Fig. 2c ist gezeigt, dass mittels der Verfahren der Spektrennormierung 27, beispielsweise mittels Vektor- oder Flächennormierung, sich Variationen in der Fluoreszenzintensität 21 (kleiner Kreis) kompensieren und die zufälligen Einflüsse physikalischer Parameter 23 (kleiner Kreis) minimieren lassen. Damit lässt sich vorzugsweise das Fluoreszenzprofil 22 (großer Kreis) hervorheben. Gleichzeitig werden nur wenige Informationen der molekularen Zusammensetzung 20 (kleiner Kreis), beruhend auf den Ramansignalen, minimiert. Da das Fluoreszenzprofil 22, also die spektrale Charakteristik der Fluoreszenz, durch die molekulare Zusammensetzung 20 bestimmt wird, lassen sich geschlechtsrelevante Informationen hervorheben.

In Fig. 2d ist gezeigt, dass eine möglichst vollständige Korrektur des sogenannten Untergrundes von Ramanspektren 28 zur alleinigen Hervorhebung der Ramanbanden, also der Informationen zur molekularen Struktur und Zusammensetzung 20 (großer Kreis) des untersuchten Objektes 2 führt.

In den Fig. 3a, 3b, 3c und 3d sind jeweils eine schematische Darstellung der den einzelnen Klassifizierungsverfahren zugeordneten Spektren (bezogen auf die relative Wellenzahl) in Bezug auf die Fig. 2a, 2b, 2c, 2d angegeben.

**Dabei wird** zumindest die Spektrenvorbehandlung 5 mit gleich bestimmten Merkmalen zumindest einer der Spektrenvorbehandlungen 6, 7, 8 mit unterschiedlich bestimmten Merkmalen zur Auswertung hinzugefügt.

In Fig. 4b ist ein Flussdiagramm für das erfindungsgemäße multiple Klassifizierungsverfahren mit Trainingsset und Testset in örtlicher Trennung, aber in algorithmischer Verbindung mit einer Klassifikationsergebnis-Gestaltung aus einer großen Anzahl von Klassifikatoren gezeigt. Dabei ist die jeweilige Spektrenvorbehandlung dabei so strukturiert, dass jeweils bestimmte Merkmale deutlicher hervortreten und andere Merkmale stärker unterdrückt werden. Die so vorbehandelten Spektren 4 werden gemäß Fig. 4b nun als variables Trainingsset 19 ausgelegt, wobei mehrere Serien 13, 14, 15, 16 von Klassifikatoren z.B. im Detail einer Serie 131, 132, 133, 134, 135 usw. berechnet werden. Üblicherweise werden alle Klassifikatoren der Serien 13, 14, 15, 16 iterativ berechnet und validiert. Variabilität bedeutet, dass ohne weitere Vorbedingungen beliebige Spektren für jeden zu berechnenden Klassifikator ausgewählt werden können. Auf diese Art lassen sich z.B. mehrere Klassifikatoren 131, 132, 133, 134, 135 für die erste Serie 13 usw. bestimmen. Für die anderen Serien 14, 15, 16 gilt dies ebenso. Die ausgesuchten Spektren des Testsets 29, z.B. 30%, werden gemäß Fig. 4b nachfolgend mit allen Klassifikatoren zu einem klassifizierten Testset 24 klassifiziert. Die Einordnung der Spektren 4; 25, 26, 27, 28 in eine bestimmte Klasse der Merkmale (männlich, weiblich) geschieht dabei vorzugsweise als Punkt-Wert (engl. Score) oder in einem Ausdruck einer Wahrscheinlichkeit zur Klassenzugehörigkeit. Um aus den Klassifikatoren der Serien 13, 14, 15, 16 bzw. 131, 132 133, 134, 135 usw. eine alleinige Aussage zu gewinnen, werden die Verhältnisse innerhalb der Klassifikatoren 13, 14, 15, 16; 131, 132, 133, 134, 135 ermittelt. Ein einfaches Mittel hierzu ist die Berechnung des Medians 30 oder, wie z.B. vordem angegeben, die Durchführung einer Clusteranalyse.

In dem in Fig. 4b dargestellten Flussdiagramm erfolgt in dem mit dem Bezugszeichen 45 = ① versehenen Punkt ein Vergleich jedes klassifizierten Spektrums jeder Form der Vorbehandlung mit dem Merkmal. Dabei wird als Ergebnis nur "richtig" oder "falsch" ausgegeben.

Beispiel:

Das Trainingset 19 umfasst 100 Spektren. Davon werden 60 für die Berechnung der Klassifikatoren ausgewählt. Werden vier Verfahren der Datenvorbehandlung 5, 6, 7, 8 eingesetzt, liegen 60 x 4 = 240 klassifizierte Spektren vor. Aus dem Vergleich mit der Merkmalsliste ergeben sich somit 240 Aussagen entweder "richtig" oder "falsch". Dieses Ergebnis wird beispielsweise in der festgelegten 129.
Iterationsstufe erzielt.

In dem mit dem Bezugszeichen 46 = ② bezeichneten Punkt erfolgt eine Bewertung der klassifizierten Spektren hinsichtlich eines festgelegten Kriteriums oder mehrerer festgelegter Kriterien. Als Kriterien dienen zum Beispiel eine Schranke der Genauigkeit oder eine maximale Anzahl iterativer Schritte. Die Kriterien können logisch UND oder logisch ODER verknüpft sein.

Beispiel: Von den möglichen 240 Aussagen sind 205 "richtig" und 35 "falsch". Damit ergibt sich eine Richtigkeit für das Trainingset von 85%.

Vor Beginn der Klassifizierung werden als Kriterien festgelegt:

1. Richtigkeit > 80% und
2. eine maximale Anzahl von Iterationen: 1000.
d.h: nach der beendeten
129. Iterationsstufe < 1000
und bei
einer erzielten Richtigkeit von 85% > vorgegebene Richtigkeit.

Bei logisch UND kann *"schlecht"* (wobei aber die Klassifikatoren als bestes Zwischenergebnis gespeichert werden) und
bei logisch ODER kann *"gut"* erreicht werden.

**Wenn an der** mit dem Bezugszeichen 47 = ⑤ angegebenen Stelle die Anzahl der vorgegebenen, zu bestimmenden Klassifikatoren erreicht ist, werden alle Klassifikatoren (und zwar jeweils die zu dem besten Ergebnis bei Punkt ② = 45 geführt haben) an die Validierung des gesamten Trainingsets 19 übergeben. Beispiel: Es wird vorgegeben, 30 Klassifikatoren je Datenvorbehandlung 5, 6, 7, 8 zu berechnen und zu einer multiplen Klassifikation geführt. Damit werden 30 x 4 = 120 Klassifikatoren zur Validierung übergeben.

An dem mit dem Bezugszeichen 48 = ③ angegebenen Punkt/Vergleichsstelle erfolgt eine gemeinsame Bewertung der Klassifizierung von allen Spektren des Trainingsets 19 nach der Methode Leave-one-out oder Cross-Validierung.

Bei "bestandenem Test" werden die Klassifikatoren zur Klassifizierung der "unbekannten" Spektren des Testsets 29 übergeben.

Wird der Test nicht bestanden, ist keine Klassifizierung entsprechend den vorgegebenen Kriterien möglich.

In dem mit dem Bezugszeichen 49 = ④ angegebenen Punkt/Vergleichsstelle wird eine abschließende Bewertung der Klassifizierung der Spektren des Testsets 24 anhand deren bekannter Merkmale durchgeführt.

Beispiel:

Das Testset 29 umfasst 50 Spektren. Diese wurden jeweils mit 120 Klassifikatoren klassifiziert, d.h. jedem Spektrum werden 120 Wahrscheinlichkeiten für die Klassenzugehörigkeit zugeordnet. Entsprechend des Medians oder der Clusteranalyse ergibt sich daraus die Zugehörigkeit zu einer Klasse. Dies ist das Ergebnis der multiplen Klassifizierung für jedes einzelne Spektrum. Sind von den 50 Spektren beispielsweise 41 richtig klassifiziert, ergibt sich daraus eine Richtigkeit für das gesamte Testset 24 von 82%,

Aus dem Vergleich mit der Merkmalsliste wird das so erstellte Verfahren 1 der multiplen Klassifikation abschließend bewertet. Damit ist das Verfahren erstellt und kann nun für Spektren ohne Kenntnis der Merkmale eingesetzt werden.

In Fig. 5 ist eine schematische Darstellung einer Wahrscheinlichkeits/Klassifikatorenanzahl-Säulendarstellung 38 für 20 Klassifikatoren gemäß Fig. 1 für die Anzeige eines als weiblich identifizierten Eies 2 gezeigt. Dabei können die unschraffierten Endbereiche/Stirnflächen 33 von Säulen 34 als dem weiblichen Geschlecht zugehörig oberhalb einer bestimmten Linienführung - der Trenngrenze 42 - liegen und die Säulen 35 mit den schraffierten Endbereichen/Stirnflächen 36 als dem männlichen Geschlecht zugehörig unterhalb der Trenngrenze 42 liegen. Der Trenngrenzenwert liegt in Fig. 5 bei 0,5 und der Median 30 hat dort den Wert 0.72. Damit wird das Ei 2 als eindeutig "weiblich" eingeordnet.

In Fig. 6 ist die zur perspektivischen Säulendarstellung zugehörige Draufsicht gezeigt, die auf einem Farb-Display als Klassifikationsergebnisbild 37 mit der Mehrzahl der unschraffierten Endbereiche/Stirnflächen 33 für die Objektinformation 31 "weiblich" angegeben ist. Auf dem Farb-Display können die unschraffierten Stirnflächen 33 rot und die schraffierten Stirnflächen 36 blau ausgebildet sein, so dass auch eine farbliche sichtbare Darstellung der Bewertung des Geschlechts gemacht werden kann.

Dabei können die schraffierten Quadrate in blauer Farbe und die unschraffierten Quadrate in roter Farbe dargestellt sein. Die wenigen blauen Quadrate zeigen die männliche Objektinformation 32 an. Die überwiegend roten Quadrate zeigen die weibliche Objektinformation 31 an. Da die roten Quadrate überwiegen, kann das Geschlecht des bebrüteten Hühnereies 2 als weibliches Merkmal 31 identifiziert werden.

In Fig. 7 ist eine Darstellung des berechneten Medians 30 bei 10 Klassifikatoren im Verhältnis zur Anzahl der zwanzig Klassifikatoren für ein Ei 2 gemäß Fig. 1 mit einer Serie von je fünf Klassifikatoren 131, 132, 133, 134, 135 pro Gruppe bei vier Gruppen 9, 10, 11, 12 angegeben. Dabei ergeben in der Säulen- und Median-Darstellung 17 Klassikatoren die Anzeige eines weiblichen Eies 2. Das Gesamtklassifikationsergebnis 18 kann mit dem berechneten Median 30 angegeben werden.

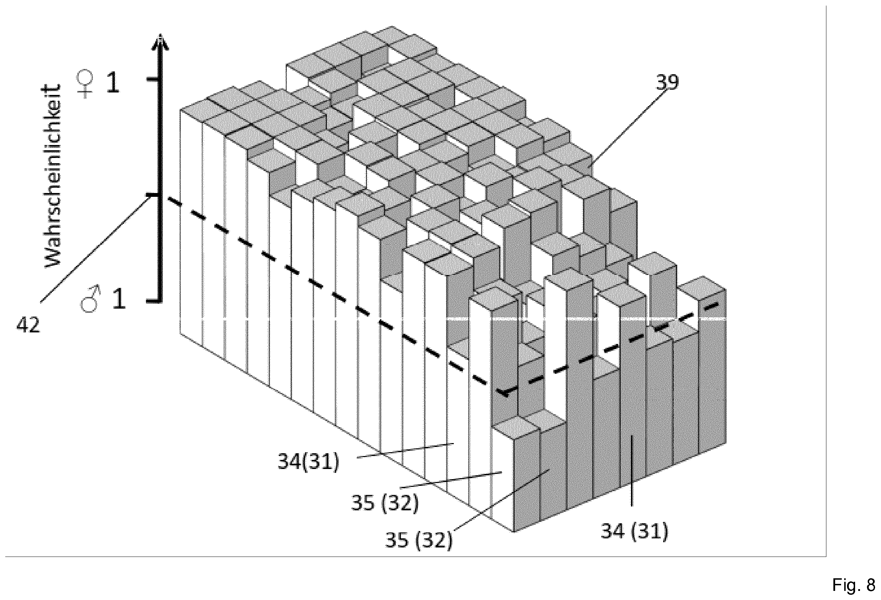

In Fig. 8 ist eine schematische Darstellung einer weiteren beispielshaften Wahrscheinlichkeits/Klassifikatorenanzahl-Säulendarstellung 39 für 120 Klassifikatoren, als Säulen dargestellt, für die Anzeige eines als weiblich identifizierten Eies 2 gezeigt. Die Trenngrenze 42 zeigt auch hier die Grenze zwischen dem Merkmal "männlich" und dem Merkmal "weiblich". Auch hier sind die oberhalb der Trenngrenze 42 endenden Säulen 34 (31) auf den Stirnflächen unschraffiert und die unterhalb der Trenngrenze 42 endenden Säulen 35 (32) schraffiert angegeben.

Bei einem Ei 2 mit männlichem Geschlechtsmerkmal 32 kann eine andere Säulendarstellung ausgebildet sein, wobei dann die oberhalb der Trenngrenze 42 liegenden Stirnflächen der ausgebildeten Säulen 35 in ihrer Mehrzahl gegenüber den nicht schraffierten Stirnflächen der Säulen 34 schraffiert sind (nicht gezeichnet).

In Fig. 9 ist eine Darstellung des berechneten Medians 30 im Verhältnis zur Anzahl der insgesamt 120 Klassifikatoren gemäß der Säulendarstellung in Fig. 8 in einer Wahrscheinlichkeits/Klassifikatoranzahl-Darstellung für ein Ei 2 mit weiblichem Geschlechtsmerkmal 31 mit nach steigenden Punkten sortierten Klassifikatoren angegeben. Der Median 30 liegt hier bei der Hälfte der ermittelten 120 Klassifikatoren und hat einen Wert der Wahrscheinlichkeit von 0,95.

Die in einer Auswerteeinheit enthaltenen Klassifizierungseinheiten/-gruppen 9, 10, 11, 12 zur Bestimmung der Objektinformation in Form von dualen Geschlechtsmerkmalen 31, 32 - weiblich oder männlich - von befruchteten und unbebrüteten und bebrüteten Eiern 2 funktionieren folgendermaßen:
Es wird die Funktionsweise erläutert.

Von jeder Klasse 25, 26, 27, 28 werden nach der Spektrenvorbehandlung 5, 6, 7, 8 mehrere Klassifikatoren der Serien 13, 14, 15, 16 berechnet. Die Bestimmung der Klassifikatoren-Serien 13, 14, 15, 16 geschieht nach einem Algorithmus der in einer Art Tandemverfahren zunächst spektrale Regionen $R_S$ aus der Koordinate der relativen Wellenzahlen auswählt und die Intensitätswerte der ausgewählten Regionen $R_S$ nachfolgend mittels Diskriminanzanalyse klassifiziert.

Im Vergleich zu den Trainingsdaten der Klassengehörigkeit erfolgt in einem wiederholten Schritt erneut eine Auswahl von Spektren-Klassen und die Klassifzierung der Intensitätswerte. Dieser Zyklus wird iterativ bis zum Erreichen einer nicht mehr verbesserbaren Genauigkeit wiederholt, wobei das Abbruchkriterium vorgegeben werden kann.

Die Gefahr des Übertrainierens und damit ein Erreichen hoher Instabilitäten wächst, je mehr spektrale Klassen 25, 26, 27, 28 für die Klassifizierung herangezogen werden. Daher ist es wünschenswert, nur wenige (3 bis maximal 20) spektrale Klassen für die Erstellung der Klassifikator-Serien 13, 14, 15, 16 einzusetzen. Da aber die geschlechtsrelevanten Informationen über den gesamten Spektralbereich, wenn auch unterschiedlich, verteilt sind, würden bei der Erstellung nur eines Klassifikators sogar wesentliche spektrale Informationen ungenutzt bleiben. Aus diesem Grund ist es zweckmäßig, dass mehrere (10 bis 30) Klassifikatoren in den Serien 13, 14, 15, 16 pro Gruppe der Datenvorbehandlung 5, 6, 7, 8 berechnet werden.

Dies hat zum Vorteil, dass zum einen die Genauigkeit der Klassifizierung verbessert wird, allein darauf beruhend, dass möglichst viele spektrale Informationen einbezogen werden und zum anderen die Robustheit, d.h. die Stabilität, vergrößert wird, da mehrere Klassifikatoren der Serien 13, 14, 15, 16 die Zuordnung tragen und einzelne Fehlzuordnungen kompensiert werden.

Die den Klassifizierungen zugeordneten Hardware-Einheiten arbeiten für alle vier Gruppen 9, 10, 11, 12 gleich. Somit kann auch anstelle der vier parallel angesteuerten Einheiten nur eine Einheit eingesetzt werden, die seriell in einer vorgegebenen Reihenfolge die Serien 13, 14, 15, 16 der Klassifikatoren erstellt. Bei der Festlegung der Anzahl der berechneten Klassifikatoren $N_G$ in den Serien 13, 14, 15, 16 je Gruppe 9, 10, 11, 12 ist der Umfang der spektralen Datenpunkte $v_S$ und die doppelte Halbbreite der spektralen Regionen $w_S$ als auch die Anzahl der ausgewählten spektralen Regionen $R_S$ zu berücksichtigen:

$$N_G = \frac{v_S}{2w_S \cdot R_S} \qquad\qquad\qquad (I)$$

Mit der Gleichung (I) wird sichergestellt, dass jeder Datenpunkt $v_S$ mit gleicher Wahrscheinlichkeit ausgewählt werden kann.

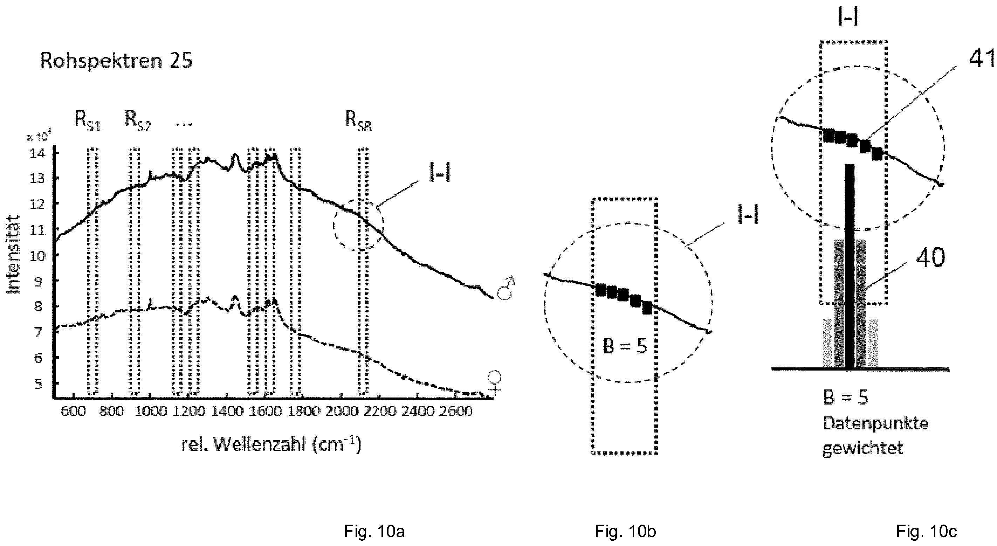

In den Fig. 10a und Fig. 10b werden am Beispiel der Rohspektren (Intensitäts/Wellenzahl-Kurven) zwanzig Klassifikatoren pro Datenvorbehandlung 25 angegeben. Dazu ist in Fig. 10a ein Ausschnitt aus den zugehörigen männlichen Intensitäts/Wellenzahl-Kurven vergrößert dargestellt. Dabei beträgt der Umfang der spek- tralen Datenpunkte $v_S$ im gesamten Spektralbereich zwischen 500 cm$^{-1}$ und 2750 cm$^{-1}$ Datenpunkte. Die Anzahl der ausgewählten spektralen Regionen $R_S$ beträgt in den Fig. 10a und Fig. 1b $R_S$ = 8 mit $R_{S1}$, $R_{S2}$, $R_{S3}$, $R_{S4}$, $R_{S5}$, $R_{S6}$, $R_{S7}$, und Rss.

Daraus können gemäß Gleichung (I) zwanzig Klassifikatoren $N_G$ für das Rohspektrum 25 berechnet werden. Das bedeutet bei vier Datenvorbehandlungen 25, 26, 27, 28 insgesamt 80 erzeugte (20 Klassifikatoren/Gruppe x 4 Gruppen) Klassifikatoren.

Nach dem vergrößerten Ausschnitt in Fig. 10c können die Datenpunkte $v_S$ auch zusätzlich gewichtet werden. Dazu ist ein Wichtungs-Diagramm 40 angegeben, aus dem erkenntlich ist, dass dem mittleren Datenpunkt 41 der höchste Wichtungswert zugeordnet ist.

Das kann sowohl mit dem männlichen Spektrum als auch mit dem weiblichen Spektrum durchgeführt werden.

Die Bewertung 17 und die Klassifizierung der den Klassifikatoren der Serien 13, 14, 15, 16 zugeordneten Ergebnisse werden in einer Bewertungseinheit durchgeführt und zu einem Klassifikationsergebnis 18 (30) geführt.

Schließlich wird ein Klassifikationsergebnis 18 in Form des Medians 30 ausgegeben, das bei der Geschlechtsbestimmung von Hühnereiern die duale Geschlechtsinformation 31, 32 (männlich oder weiblich) mit der höchsten Wahrscheinlichkeit darstellt.

Das erfindungsgemäße Verfahren kann im Allgemeinen mit folgenden Schritten im Detail absolviert werden:

- Aufnahme und Registrierung der Spektren mittels zumindest einer optischen Einrichtung mit zumindest einem Spektrometer und/oder weiteren Detektoren,
- Erzeugung von digitalisierten Signalen in Form von Datenpunkten und Speicherung der detektierten Spektren in Speichereinheiten von Klassifikationseinheiten einer Auswerteeinheit,
- Spektrenvorbehandlung, indem die gespeicherten Spektren in den einzelnen Speichereinheiten einzeln ausgewertet werden und die zugehörigen digitalisierten ausgewerteten Signale zur Weiterverarbeitung bereit gestellt werden,
- Trennung der vorbehandelten Spektren als Trainingsset und als Testset,
- Auslegung der vorbehandelten Spektren als Trainingsset und von einem vom Trainingsset getrennten Testset, wobei erfindungsgemäß zumindest eine
- Berechnung der Klassifikatoren der Serien der berücksichtigten einzelnen Klassifizierungsverfahren unter Einbeziehung von iterativen Verfahren und einer Validierung in den Klassifizierungseinheiten,
- Klassifizierung der ausgewerteten Spektren des Trainingssets mit allen Klassifikatoren der Serien,
- Einordnung der Spektren des Trainingssets in eine Klasse von Objektinformationen mit einem Ausdruck einer Wahrscheinlichkeit zur Klassenzugehörigkeit,
- Berechnung des Medians oder Durchführung einer Clusteranalyse zur Darstellung des Wahrscheinlichkeitsergebnisses der einer Klasse zugehörigen Objektinformationen des Trainingssets,
- Klassifizierung der ausgewerteten Spektren des Testsets mit allen Klassifikatoren der Serien,
- Einordnung der Spektren des Testsets in eine Klasse von Objektinformationen mit einem Ausdruck einer Wahrscheinlichkeit zur Klassenzugehörigkeit und
- Berechnung des Medians oder eine Durchführung einer Clusteranalyse zur Darstellung des Wahrscheinlichkeitsergebnisses/Klassifikationsergebnisses der einer Klasse zugehörigen Objektinformationen des Testsets

durchgeführt werden.

Zusätzlich ist zu bemerken, dass im Allgemeinen die registrierten Spektren 4 naturgemäß hochvariabel sind. Dies begründet sich zum einen in der immanenten Variabilität biologischer Systeme und zum anderen in der Sensitivität der ramanspektroskopischen Messungen. Externe Störungen systematischer und zufälliger Art führen zu einer hohen Variabilität der spektralen Merkmale und überlagern somit die merkmalsrelevanten Informationen. Weiterhin tritt bei dem Verfahren der Ramanspektroskopie auch Fluoreszenzlicht auf, das zwar ebenso molekulare Informationen enthält, jedoch gleichzeitig die in der Regel viel schwächeren Ramanspektroskopischen molekularen Informationen über die Zusammensetzung des untersuchten Objektes 2 überlagert.

Anhand dieser Vorbemerkung und der Fig. 1 soll ein weiteres Ausführungsbeispiel mit mehr als zwei Merkmalen von Objektinformationen erläutert werden. In Fig. 1 ist in der schematischen Block-Darstellung ein erfindungsgemäßes Verfahren 1 zur Klassifizierung von Spektren 4 eines Objektes 2 mit komplexem Informationsgehalt, insbesondere von optischen Molekülspektren 4 zur Zuordnung von Objektinformationen/Merkmale 3 für eine wahrscheinliche Bestimmung einer beispielsweisen dualen Objektinformation 31, 32 oder von vier Objektinformationen 51, 52, 53, 54 gezeigt.

Als zu untersuchende Objekte 2 können auch Gewebeproben, beispielsweise Hirntumore, benutzt und eingesetzt werden und anstelle der dualen Merkmalsinformationen 31, 32 können beispielsweise auch vier unterschiedliche Merkmale 3 mit 51, 52, 53, 54 ausgewählt und bestimmt werden, beispielsweise

> Merkmal 51: gesundes Gewebe,
> Merkmal 52: Tumorgewebe mit Tumorgrad I und II entsprechend dem histolgischen Gradierungsschema der Weltgesundheitsorganisation (WHO),
> Merkmal 53: Tumorgewebe mit Tumorgrad III und IV entsprechend WHO und
> Merkmal 54: nekrotisches Gewebe.

Die Aufnahme und Registrierung der Rückstreustrahlung von der Gewebeprobe aus erfolgt mittels zu mindestens einer optischen Einrichtung beispielsweise wie in der Druckschrift DE 10 2014 010 150 A1 beschrieben. Die registrierten Rückstreuspektren 4 werden digitalisiert und in einer Auswerteeinheit gespeichert. Die Datenvorbehandlung erfolgt durch beispielsweise drei verschiedene Verfahren 5, 6, 7, die dabei erhaltenen Datensätze können beispielsweise jeweils Rohspektren, normierte Spektren und Spektren mit einer nichtlinearen Basislinienkorrektur enthalten, wobei die gespeicherten Spektren in den einzelnen Speichereinheiten einzeln ausgewertet und die zugehörigen digitalisierten ausgewerteten Signale zur Weiterverarbeitung bereit gestellt werden. Die vorbehandelten Spektren werden als Trainingsset ausgelegt, wobei erfindungsgemäß eine Berechnung der Klassifikatoren der Serien der berücksichtigten einzelnen Klassifizierungsverfahren unter Einbeziehung von iterativen Verfahren und einer Validierung in den Klassifizierungseinheiten durchgeführt wird. Es erfolgt weiterhin die Klassifizierung der ausgewerteten Spektren des Testsets mit allen Klassifikatoren der Serien und die Einordnung der Gewebespektren in eine Klasse von Objektinformationen entsprechend den Merkmalen 51 bis 54 mit einem Ausdruck einer Wahrscheinlichkeit zur Klassenzugehörigkeit. Die Klassifizierung wird mit der Berechnung des Medians oder mittels einer Clusteranalyse bewertet und das Wahrscheinlichkeitsergebnis/Klassifikationser- gebnisses der einer Klasse zugehörigen Objektinformationen des Testsets dargestellt. Das heißt, dass für jedes registrierte Spektrum einer Gewebeprobe mittels multipler Klassifikation ein Punktwert berechnet wird, der nach festgelegten Trenngrenzen in einem der 4 Wahrscheinlichkeitsbereiche liegt, die dem histologischen Befund der Merkmale: 51 - Gesund / 52 - WHO I,II / 53 - WHO III,IV / 54 - Nekrose entsprechen.

Eine Vorrichtung zur Klassifizierung von Spektren 4 von Objekten 2 mit komplexem Informationsgehalt, vorzugsweise mit Objekten in Form von Hühnereiern 2 für eine Bestimmung einer dualen Eiinformation 31, 32 - weiblich oder männlich -, in der das vorgenannte Verfahren realisiert wird und die weitgehend der Block(Box)-Darstellung in Fig. 1 entsprechend ausgebildet ist, kann zumindest folgende Einheiten umfassen

- zumindest eine detektierende optische Einrichtung mit zumindest einem Spektrometer und/oder weitere Detektoren zur Aufnahme und Registrierung der Spektren 4,
- eine Einheit zur Erzeugung von digitalisierten Signalen in Form von Datenpunkten, die die Spektren 4 realisieren.
- Speichereinheiten zur Speicherung der Spektren 4 in den Klassifikationseinheiten/-gruppen 9, 10, 11, 12 einer die Klassifikationseinheiten umfassenden Auswerteeinheit,
- Einheiten zur Spektrenvorbehandlung 5, 6, 7, 8, in denen die gespeicherten Spektren 4 in den einzelnen Speichereinheiten einzeln ausgewertet werden und die zugehörigen digitalisierten ausgewerteten Signale zur Weiterverarbeitung bereitgestellt werden,
- Trainingssets 19 zur Auslegung und Einsetzung der vorbehandelten Spektren 4; 25, 26, 27, 28,
- mindestens eine Klassifizierungseinheit für die Gruppen 9, 10, 11, 12 zur Berechnung der Klassifikatoren der Serien 13, 14, 15, 16 der berücksichtigten einzelnen herkömmlichen Klassifizierungsverfahren 25, 26, 27, 28 unter Einbeziehung von iterativen Verfahren und einer Validierung in den Klassifizierungseinheiten,
- Testsets 29 zur Klassifizierung der ausgewerteten Spektren 4 mit allen Klassifikatoren der Serien 13, 14,1 5, 16,
- eine Einheit zur Einordnung der Spektren 4 in beispielsweise eine duale Klasse - männlich 32 oder weiblich 31 - von Objekt(Ei)informationen mit einem Ausdruck einer Wahrscheinlichkeit zur Klassenzugehörigkeit,

- eine Bewertungseinheit zur Berechnung des Klassifikationsergebnisses 18 z.B. in Form des Medians 30 oder nach Durchführung einer Clusteranalyse zur Darstellung des Wahrscheinlichkeitsergebnisses der beispielsweise einer der dualen Klasse - weiblich oder männlich - zugehörigen Objekt(Ei)information 31, 32.

Eine gleiche Vorrichtung kann für das multiple Klassifizierungsverfahren mit den vier Merkmalen 51, 52, 53, 54 oder mit weiteren vorgegebenen Merkmalen aufgebaut werden.

Bezugszeichenliste

| | |
|---|---|
| 1 | Verfahren in einer Box-Darstellung |
| 2 | Objekt/Ei |
| 3 | Objektinformationen/Merkmale |
| 4 | registrierte Spektren |
| 5, 6, 7, 8 | Vorbehandlung |
| 9, 10, 11, 12 | Klassifizierung |
| 13, 14, 15, 15, 16 | Serie von Klassifikatoren |
| 131, 132, 133,134, 135, 136 | Klassifikator |
| 17 | Bewertung |
| 18 | Klassifikationsergebnis |
| 19 | Trainingsset |
| 20 | Molekulare Zusammensetzung |
| 21 | Fluoreszenzintensität |
| 22 | Fluoreszenzprofil |
| 23 | Variation physikalische Parameter |
| 24 | Klassifiziertes Testset |
| 25, 26, 27, 28 | vorbehandelte Spektren |
| 29 | Testset mit vorzugsweise ausgewählten 30% der Spektren |
| 30 | Median |
| 31 | Objektinformation/Merkmal weiblich |
| 32 | Objektinformation/Merkmal männlich |
| 33 | unschraffierte Stirnfläche, dem weiblichen Geschlecht zugeordnet |
| 34 | Säule des weiblichen Geschlechts |
| 35 | Säule des männlichen Geschlechts |
| 36 | Schraffierte Stirnfläche, dem männlichen Geschlecht zugeordnet |
| 37 | Klassifikationsergebnisbild |
| 38, 39 | Säulendarstellung |
| 40 | Wichtungs-Diagramm |
| 41 | Mittlerer Datenpunkt in der Region einer Spektrumskurve |
| 42 | Festgelegte Trenngrenze |
| 43 | erstes Histogramm der Clusteranalyse |
| 44 | zweites Histogramm der Clusteranalyse |
| 45 | = ① Vergleichspunkt |
| 46 | = ② Vergleichspunkt |
| 47 | = ⑤ Vergleichspunkt |
| 48 | = ③ Vergleichspunkt |
| 49 | = ④ Vergleichspunkt |
| 50 | Klassifikator nach dem Stand der Technik |
| 51, 52, 53, 54 | Merkmal |

**Patentansprüche**

1. Verfahren zur Klassifizierung von Spektren von Objekten mit komplexem Informationsgehalt mit mindestens zwei unterschiedlichen Objektinformationen unter Einsatz von Verfahren zur Registrierung und Vorbehandlung von spektralen Daten und eines der Datenvorbehandlung zugehörigen Verfahrens der Klassifizierung mit der Berechnung eines Klassifikators,

wobei ein multiples Klassifizierverfahren mit mindestens zwei unterschiedlichen Verfahren der Datenvorbehandlung der Spektren und des der jeweiligen Datenvorbehandlung zugeordneten Verfahrens zur Klassifizierung durchgeführt wird, und folgende Schritte durchgeführt werden:

- Aufnahmen und Registrieren der Spektren mittels zumindest einer optischen Einrichtung mit zumindest einem Spektrometer und/oder weiteren Detektoren,
- Durchführen einer Spektrenvorbehandlung, in dem die registrierten Spektren einzeln vorbehandelt werden und die zugehörigen digitalisierten ausgewerteten Signale zur Weiterverarbeitung bereitgestellt werden,
- Trennen der vorbehandelten Spektren als Trainingsset und als Testset
- Berechnen mehrerer Klassifikatoren pro Art der Datenvorbehandlung durch iterative Berechnung und Validierung ausgehend vom Trainingsset, indem spektrale Regionen $R_S$ aus einer Koordinate von relativen Wellenzahlen ausgewählt werden und die Intensitätswerte der ausgewählten Regionen $R_S$ nachfolgend mittels Diskriminanzanalyse klassifiziert werden, wobei in einem wiederholten Schritt eine erneute Auswahl spektraler Regionen $R_S$ und die Klassifizierung der Intensitätswerte erfolgt, und wobei der Zyklus iterativ bis zum Erreichen einer nicht mehr verbesserbaren Genauigkeit unter Vorgabe eines Abbruchkriteriums wiederholt wird,
- Klassifizieren der vorbehandelten Spektren des Testsets mit allen ausgehenden vom Trainingsdatenset berechneten Klassifikatoren,
- Einordnen der Spektren in eine Klasse von Objektinformationen mit einem Ausdruck einer Wahrscheinlichkeit zur Klassifikationszugehörigkeit,
- Berechnen eines Klassifikationsergebnisses durch Berechnen eines Medians oder durch Durchführen einer Clusteranalyse zur Darstellung des Wahrscheinlichkeitsergebnisses der einer Klasse zugehörigen Objektinformationen.

2. Verfahren nach Anspruch 1, bei dem bei der Festlegung der Anzahl der berechneten Klassifikatoren $N_G$ in den Serien je Klassifizierungsgruppe der Umfang der spektralen Datenpunkte ($v_S$) sowie die doppelte Halbbreite der spektralen Regionen $w_S$ als auch die Anzahl der ausgewählten spektralen Regionen ($R_S$) berücksichtigt werden:

$$N_G = \frac{v_S}{2w_S \cdot R_S}$$

(I)

wobei mit der Gleichung (I) sichergestellt wird, dass jeder Datenpunkt mit gleicher Wahrscheinlichkeit ausgewählt werden kann.

3. Verfahren nach Anspruch 2, bei dem die zu dem Umfang der spektralen Datenpunkte $v_S$ gehörenden Datenpunkte gewichtet werden.

4. Verfahren nach Anspruch 1, bei dem für zumindest eine der Spektrenvorbehandlungen Rohspektren, Basislinienkorrekturen , Normierungen, Ableitungen, Covarianz und/oder eine Hauptkomponentenanalyse eingesetzt werden, sodass jeweils bestimmte Merkmale hervortreten und andere bestimmte Merkmale unterdrückt werden.

5. Verfahren nach Anspruch 4, bei dem zumindest eine Spektrenvorbehandlung mit gleich bestimmten und gleich gewichteten Merkmalen zumindest einer der Spektrenvorbehandlungen mit unterschiedlich bestimmten Merkmalen der Auswertung hinzugefügt wird.

6. Verfahren nach Anspruch 1, bei dem die vorbehandelten Spektren als Trainingsset ausgelegt und mehrere Klassifikatoren der Serien oder Klassifikatoren bestimmt und validiert werden.

7. Verfahren nach Anspruch 1, bei dem als ein Verfahren zur Klassifizierung in den Klassifizierungsgruppen ein Verfahren der neuronalen Netze und/oder ein Verfahren der linearen Zeit-Frequenz-Transformationen (engl. Wavelets) eingesetzt werden.

8. Verfahren nach Anspruch 1, bei dem vorbehandelte Spektren der optischen Molekülspektroskopie, vorzugsweise Absorption, Emission, Streuung oder UV/vis, NIR, IR Absorption, Fluoreszenz, Raman, klassifiziert werden.

9. Verfahren nach den Ansprüchen 1 bis 8, bei dem als Objekte wahlweise Vogeleier, vorzugsweise Hühnereier eingesetzt werden und als Objektinformationen die duale Information über das weibliche Eigeschlecht und über das männliche Eigeschlecht benutzt werden.

10. Verfahren nach den Ansprüchen 1 bis 9, bei dem als Objekte wahlweise Gewebeproben, beispielsweise Hirntumore, eingesetzt werden und als Objektinformationen vier Merkmale benutzt und ausgewählt sowie bestimmt werden, und zwar:

- gesundes Gewebe,
- Tumorgewebe mit Tumorgrad I und II entsprechend dem histolgischen Gradierungsschema der Weltgesundheitsorganisation,
- Tumorgewebe mit Tumorgrad III und IV entsprechend WHO und
- nekrotisches Gewebe.

11. Verfahren nach Anspruch 1, bei dem nach einem Durchlauf mindestens zweier mit je einer vorangegangenen spektrumunterschiedlichen Datenvorbehandlung versehenen Klassifizierungsverfahren mit mindestens einem ermittelten Klassifikator gesamtheitlich mindestens zwei ermittelte Klassifikatoren zur Bewertung und der nachfolgenden Ermittlung eines Wahrscheinlichkeitsergebnisses bezüglich der vorgegebenen unterschiedlichen Objektinformationen erreicht und eingesetzt werden, wobei das Wahrscheinlichkeitsergebnis ausgegeben wird, so dass ein Schluss zumindest auf die mit einem höchsten Wert ermittelte Objektinformation ermöglicht wird.

12. Vorrichtung zur Klassifizierung von Spektren von Objekten mit komplexem Informationsgehalt, in der das vorgenannte Verfahren nach einem der Ansprüchen 1 bis 11 realisiert wird, umfassend zumindest folgende Einheiten

- zumindest eine detektierende optische Einrichtung mit zumindest einem Spektrometer und/oder weitere Detektoren zur Aufnahme und Registrierung der Spektren,
- eine Einheit zur Erzeugung von digitalisierten Signalen in Form von Datenpunkten, die die Spektren realisieren,
- Speichereinheiten zur Speicherung der registrierten Spektren in den Klassifikationseinheiten/-gruppen einer die Klassifikationseinheiten umfassenden Auswerteeinheit,
- Einheiten zur Spektrenvorbehandlung, in denen die registrierten Spektren einzeln vorbehandelt werden und die zugehörigen digitalisierten ausgewerteten Signale zur Weiterverarbeitung bereitgestellt werden,
- Trainingssets zur Auslegung und Einsetzung der vorbehandelten Spektren,
- Mindestens eine Klassifizierungseinheit für die Berechnung mehrerer Klassifikatoren pro Art der Datenvorbehandlung durch einer iterative Berechnung und einer Validierung ausgehend vom Trainingsset in den Klassifizierungseinheiten, bei der spektrale Regionen $R_s$ aus einer Koordinate von relativen Wellenzahlen auswählbar sind und die Intensitätswerte der ausgewählten Regionen $R_s$ nachfolgend mittels Diskriminanzanalyse klassifizierbar sind, wobei in einem wiederholten Schritt eine erneute Auswahl spektraler Regionen $R_s$ und die Klassifizierung der Intensitätswerte realisierbar ist, und der Zyklus iterativ bis zum Erreichen einer nicht mehr verbesserbaren Genauigkeit unter Vorgabe eines Abbruchkriteriums wiederholbar ist,
- Testsets zur Klassifizierung der vorbehandelten Spektren mit allen Klassifikatoren pro Art der Datenvorbehandlung,
- eine Einheit zur Einordnung der vorbehandelten Spektren in mindestens eine duale Klasse von Objektinformationen mit einem Ausdruck einer Wahrscheinlichkeit zur Klassenzugehörigkeit,
- eine Bewertungseinheit zur Berechnung des Klassifikationsergebnisses in Form des Medians oder Durchführung einer Clusteranalyse zur Bestimmung des Wahrscheinlichkeitsergebnisses zumindest einer der vorgegebenen Objektinformationen.

**Claims**

1. Method for classifying spectra of objects having complex information content with at least two different pieces of object information, using a method for recording and pretreating spectral data and a classification method associated with the data pretreatment, which classification method includes the calculation of a classifier, wherein a multiple classification method with at least two different methods for the data pretreatment of the spectra and for the classification method assigned to the corresponding data pretreatment is carried out, and the following steps are carried out:

- capturing and recording the spectra by means of at least one optical device comprising at least one spectrometer and/or further detectors,
- carrying out a spectra pretreatment, in which the recorded spectra are individually pretreated and the associated digitized evaluated signals are provided for further processing,

- separating the pretreated spectra as a training set and as a test set
- calculating a plurality of classifiers per type of data pretreatment through iterative calculation and validation on the basis of the training set by spectral regions $R_S$ being selected from a coordinate of relative wave numbers and the intensity values of the selected regions $R_S$ being subsequently classified by means of discriminant analysis, wherein a renewed selection of spectral regions $R_S$ and the classification of the intensity values take place in a repeated step, and the cycle is repeated iteratively until an accuracy that can no longer be improved is achieved, subject to an abortion criterion,
- classifying the pretreated spectra of the test set with all classifiers that are calculated on the basis of the training data set,
- sorting the spectra into a class of object information with an expression of a probability of belonging to the classification,
- calculating a classification result by calculating a median or by carrying out a cluster analysis in order to represent the probability result of the piece of object information belonging to a class.

2. Method according to claim 1, in which the scope of the spectral data points ($v_S$) and twice the half-width of the spectral regions $w_S$ as well as the quantity of selected spectral regions ($R_S$) are taken into account when determining the quantity of calculated classifiers $N_G$ in the series per classification group:

$$N_G = \frac{v_S}{2w_S \cdot R_S} \qquad\qquad (1)$$

wherein equation (I) ensures that each data point can be selected with equal probability.

3. Method according to claim 2, in which the data points belonging to the scope of the spectral data points vs are weighted.

4. Method according to claim 1, in which raw spectra, baseline corrections, normalizations, derivations, covariance and/or principal-component analysis are used for at least one of the spectra pretreatments, such that in each case defined features are emphasized and other defined features are suppressed.

5. Method according to claim 4, in which at least one spectra pretreatment having equally defined and equally weighted features is added to at least one of the spectra pretreatments having differently defined features of the evaluation.

6. Method according to claim 1, in which the pretreated spectra are designed as a training set and a plurality of classifiers of the series or classifiers are defined and validated.

7. Method according to claim 1, in which a method relating to neural networks and/or a method relating to linear time-frequency transformations (wavelets) is used as a classification method in the classification groups.

8. Method according to claim 1, in which pretreated spectra of optical molecular spectroscopy, preferably absorption, emission, scattering or UV/vis, NIR, **IR** absorption, fluorescence, Raman, are classified.

9. Method according to claims 1 to 8, in which optionally bird eggs, preferably chicken eggs, are used as objects and the dual information items about the female egg sex and the male egg sex are used as pieces of object information.

10. Method according to claims 1 to 9, in which optionally tissue samples, for example brain tumors, are used as objects and four features are used, selected and determined as pieces of object information, namely:

- healthy tissue,
- tumor tissue having tumor grade I and II according to the histological grading schema of the World Health Organization,
- tumor tissue having tumor grade **III** and IV according to WHO and
- necrotic tissue.

11. Method according to claim 1, in which, after at least two classification methods have been performed with at least one determined classifier, each classification method having a preceding spectrum-different data pretreatment, overall at least two determined classifiers for evaluating and subsequently determining a probability result with respect to the predefined different pieces of object information are obtained and used, wherein the probability result is output, so that it is possible to make a conclusion at least about the piece of object information determined to have the highest value.

12. Device for classifying spectra of objects having complex information content, in which the aforementioned method according to any of claims 1 to 11 is implemented, comprising at least the following units

   - at least one detecting optical device comprising at least one spectrometer and/or further detectors for capturing and recording the spectra,
   - a unit for generating digitized signals in the form of data points that realize the spectra,
   - storage units for storing the recorded spectra in the classification units/groups of an evaluation unit that comprises the classification units,
   - spectra pretreatment units in which the recorded spectra are individually pretreated and the associated digitized evaluated signals are provided for further processing,
   - training sets for the interpretation and use of the pretreated spectra,
   - at least one classification unit for calculating a plurality of classifiers per type of data pretreatment through an iterative calculation and a validation based on the training set in the classification units, in which unit spectral regions Rs are selectable from a coordinate of relative wave numbers and the intensity values of the selected regions $R_S$ are subsequently classifiable by means of discriminant analysis, wherein a renewed selection of spectral regions Rs and the classification of the intensity values are implementable in a repeated step, and the cycle is iteratively repeatable until an accuracy that can no longer be improved is achieved, subject to an abortion criterion,
   - test sets for classifying the pretreated spectra with all classifiers per type of data pretreatment,
   - a unit for sorting the pretreated spectra into at least one dual class of pieces of object information with an expression of a probability for belonging to the class,
   - a unit of evaluation for calculating the classification result in the form of the median or carrying out a cluster analysis in order to determine the probability result of at least one of the predefined pieces of object information.

## Revendications

1. Procédé pour la classification de spectres d'objets comportant un contenu d'informations complexe comportant au moins deux informations d'objets différentes en utilisant des procédés d'enregistrement et de prétraitement de données spectrales et un procédé de classification associé au prétraitement de données avec le calcul d'un classificateur,
   dans lequel
   un procédé de classification multiple est réalisé avec au moins deux procédés différents de prétraitement de données des spectres et le procédé associé au prétraitement de données respectif pour la classification, et les étapes suivantes sont réalisées :

   - réception et enregistrement des spectres au moyen d'au moins un dispositif optique comportant au moins un spectromètre et/ou d'autres détecteurs,
   - réalisation d'un prétraitement de spectres, lors duquel les spectres enregistrés sont prétraités individuellement et les signaux évalués numérisés associés sont fournis pour un traitement ultérieur,
   - séparation des spectres prétraités en tant qu'ensemble d'apprentissage et ensemble de test
   - calcul de plusieurs classificateurs par type de prétraitement de données par calcul itératif et validation à partir de l'ensemble d'apprentissage, en sélectionnant des régions spectrales $R_s$ à partir d'une coordonnée de nombres d'ondes relatifs et en classifiant ensuite les valeurs d'intensité des régions $R_s$ sélectionnées au moyen d'une analyse discriminante, dans lequel une nouvelle sélection de régions spectrales $R_s$ et la classification des valeurs d'intensité sont effectuées au cours d'une étape répétée, et dans lequel le cycle est répété de manière itérative jusqu'à l'obtention d'une précision qui ne peut plus être améliorée en définissant un critère d'interruption,
   - classification des spectres prétraités de l'ensemble de test avec tous les classificateurs calculés provenant de l'ensemble de données d'apprentissage,
   - classement des spectres dans une classe d'informations d'objets avec une expression d'une probabilité d'association à la classification,
   - calcul d'un résultat de classification en calculant une médiane ou en réalisant une analyse par groupe pour représenter le résultat de probabilité des informations d'objets associées à une classe.

2. Procédé selon la revendication 1, dans lequel, lors de la spécification du nombre de classificateurs $N_G$ calculés dans les séries par groupe de classification, on tient compte de l'étendue des points de données spectrales ($v_S$) ainsi que de la double demi-largeur des régions spectrales $w_S$ ainsi que du nombre de régions spectrales (Rs) sélectionnées :

$$N_G = \frac{v_S}{2w_S \cdot R_S} \qquad\qquad (I)$$

dans lequel l'équation (I) garantit que chaque point de données peut être sélectionné avec la même probabilité.

3. Procédé selon la revendication 2, dans lequel les points de données appartenant à l'étendue des points de données spectrales $v_S$ sont pondérés.

4. Procédé selon la revendication 1, dans lequel, pour au moins l'un des prétraitements de spectres, des spectres bruts, des corrections de lignes de base, des normalisations, des dérivations, une covariance et/ou une analyse en composantes principales sont utilisés, de sorte que respectivement des caractéristiques déterminées sont mises en évidence et d'autres caractéristiques déterminées sont supprimées.

5. Procédé selon la revendication 4, dans lequel au moins un prétraitement de spectres comportant des caractéristiques déterminées de manière identique et pondérées de manière identique est ajouté à au moins l'un des prétraitements de spectres comportant des caractéristiques déterminées de manière différente de l'évaluation.

6. Procédé selon la revendication 1, dans lequel les spectres prétraités sont configurés comme un ensemble d'apprentissage et plusieurs classificateurs des séries ou classificateurs sont déterminés et validés.

7. Procédé selon la revendication 1, dans lequel est utilisé comme procédé pour la classification dans les groupes de classification un procédé de réseaux neuronaux et/ou un procédé de transformations linéaires temps-fréquence (en anglais « wavelets » ou « ondelettes »).

8. Procédé selon la revendication 1, dans lequel des spectres prétraités de spectroscopie moléculaire optique, de préférence d'absorption, d'émission, de diffusion ou UV-vis, proche IR, d'absorption IR, de fluorescence, Raman, sont classifiés.

9. Procédé selon les revendications 1 à 8, dans lequel sont utilisés comme objets au choix des œufs d'oiseaux, de préférence des œufs de poule, et sont utilisées comme informations d'objets les doubles informations sur le sexe femelle de l'œuf et sur le sexe mâle de l'œuf.

10. Procédé selon les revendications 1 à 9, dans lequel sont utilisés comme objets au choix des échantillons de tissu, par exemple de tumeurs cérébrales, et quatre caractéristiques sont utilisées et sélectionnées et déterminées comme informations d'objets, à savoir :

    - des tissus sains,
    - des tissus tumoraux de grade I et II selon la classification histologique de l'Organisation mondiale de la santé,
    - des tissus tumoraux de grade III et IV selon l'OMS et
    - des tissus nécrosés.

11. Procédé selon la revendication 1, dans lequel, après un passage d'au moins deux procédés de classification pourvus de respectivement un prétraitement de données préalable à spectres différents et comportant au moins un classificateur déterminé, on obtient et on utilise globalement au moins deux classificateurs définis pour l'évaluation et la définition subséquente d'un résultat de probabilité concernant les différentes informations d'objets prédéfinies, dans lequel le résultat de probabilité est délivré en sortie de sorte qu'une conclusion est possible au moins concernant les informations d'objets définies avec une valeur la plus élevée.

12. Dispositif pour la classification de spectres d'objets comportant un contenu d'informations complexe, dans lequel le procédé précité est réalisé selon l'une des revendications 1 à 11, comprenant au moins les unités suivantes

    - au moins un dispositif optique de détection comportant au moins un spectromètre et/ou d'autres détecteurs pour la réception et l'enregistrement des spectres,
    - une unité pour la génération de signaux numérisés sous forme de points de données qui réalisent les spectres,

- des unités de mémorisation pour la mémorisation des spectres enregistrés dans les unités/groupes de classification d'une unité d'évaluation comprenant les unités de classification,

- des unités pour le prétraitement de spectres, dans lesquelles les spectres enregistrés sont prétraités individuellement et les signaux évalués numérisés associés sont fournis pour un traitement ultérieur,

- des ensembles d'apprentissage pour l'interprétation et l'utilisation des spectres prétraités,

- au moins une unité de classification pour le calcul de plusieurs classificateurs par type de prétraitement de données par un calcul itératif et une validation à partir de l'ensemble d'apprentissage dans les unités de classification, dans laquelle des régions spectrales $R_s$ peuvent être sélectionnées à partir d'une coordonnée de nombres d'ondes relatifs et les valeurs d'intensité des régions $R_s$ sélectionnées peuvent ensuite être classifiées au moyen d'une analyse discriminante, dans lequel une nouvelle sélection de régions spectrales $R_s$ et la classification des valeurs d'intensité peuvent être réalisées au cours d'une étape répétée, et le cycle peut être répété de manière itérative jusqu'à l'obtention d'une précision qui ne peut plus être améliorée en définissant un critère d'interruption,

- des ensembles de test pour la classification des spectres prétraités avec tous les classificateurs par type de prétraitement de données,

- une unité pour le classement des spectres prétraités dans au moins une double classe d'informations d'objets avec une expression d'une probabilité d'association à la classe,

- une unité d'évaluation pour le calcul du résultat de classification sous la forme de la médiane ou la réalisation d'une analyse par groupe pour la détermination du résultat de probabilité d'au moins l'une des informations d'objets prédéfinies.

Fig. 1

Fig. 2a  Rohspektren 25 mit 5, 9, 13

20  molekulare Zusammen-setzung
21  Fluoreszenz-intensität
23  Variation physikalischer Parameter
22  Fluoreszenz-profil

Fig. 2b  Lineare Basislinienkorrektur 26 mit 6, 10, 14

Fluoreszenz-intensität

Fig. 2c  Normierung 27 mit 7, 11, 15

20
21
23
22  Fluoreszenz-profil

Fig. 2d  Ramanspektren 28 mit 8, 12, 16

20  molekulare Zusammen-setzung
21
22
23

Fig. 2a-2d

EP 3 513 355 B1

Fig. 3a – 3d

Bewertung des erstellten Klassifikators 50

Erstellung des Klassifikators und Klassifizierung von Spektren

50

Ergebnis: klassifizierte Spektren des Testsets

24

Klassifizierung Testset

iterative Berechnung geeigneter Klassifikator 50

29

31, 32

19

31, 32

31, 32

5

vorbehandelte Spektren

Fig. 4a

Fig. 4b

Fig. 5

Fig. 6

Fig. 7

Fig. 8

Fig. 9

Fig. 10a                    Fig. 10b                    Fig. 10c

Fig. 11a

Fig. 11c

Fig. 11b

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20120321174 A1
- WO 2010150265 A
- WO 2014021715 A2
- DE 102007013107 A1
- DE 102010006161 B3
- DE 102014010150 A1
- WO 2016000678 A1
- EP 2336751 A1
- US 6029080 B

**In der Beschreibung aufgeführte Nicht-Patentliteratur**

- **A. E. NIKULIN** ; **B. DOLENKO** ; **T., BEZABEH** ; **R. L. SOMORJAI**. Near-optimal region selection for feature space reduction: novel preprocessing methods for classifying MR spectra. *NMR Biomed.*, 1998, vol. 11 (4-5), 209-216
- **B. K. LAVINE** ; **C. E. DAVIDSON** ; **A. J. MOORES**. Genetic algorithms for spectral pattern recognition. *Vibrational Spectroscopy*, 2002, vol. 28 (1), 83-95
- **J. JACQUES** ; **C. BOUVEYRON** ; **S. GIRARD** ; **O. DEVOS** ; **L. DUPONCHEL** ; **C. RUCKEBUSCH**. Gaussian mixture models for the classification of high-dimensional vibrational spectroscopy data. *Journal of Chemometrics*, 2010, vol. 24 (11-12), 719-727
- **ELADIO RODRIGUEZ-DIAZ** ; **SATISH K. SINGH** ; **IRVING J. BIGIO** ; **DAVID A. CASTAÑÓN**. Spectral classifier design with ensemble classifiers and misclassificationrejection: application to elastic-scattering spectroscopy for detection of colonic neoplasia. *J. Biomed. Opt.*, 01 June 2011, vol. 16 (6), 067009
- **LU XU** ; **YAN-PING ZHOU** ; **LI-JUAN TANG** ; **HAI-LONG WU** ; **JIAN-HUI JIANG** ; **GUO-LI SHEN** ; **RU-QIN YU**. Ensemble preprocessing of near-infrared (NIR) spectra for multivariate calibration. *Analytica Chimica Acta*, 2008, vol. 616 (2), ISSN 0003-2670, 138-143
- **G. STEINER** ; **S. KUCHLER** ; **A. HERRMANN** ; **E. KOCH** ; **R. SALZER** ; **G. SCHACKERT** ; **M. KIRSCH**. *Cytometry*, 2008, vol. 73A, 1158-1164